(19) Europäisches Patentamt
European Patent Office
Office européen des brevets

(11) EP 1 031 349 A1

(12) **EUROPEAN PATENT APPLICATION**

(43) Date of publication:
**30.08.2000 Bulletin 2000/35**

(21) Application number: **99103723.5**

(22) Date of filing: **25.02.1999**

(51) Int Cl.[7]: **A61K 31/19**, A61K 31/40, A61K 31/53, A61K 31/435, A61K 31/24, A61K 31/425, A61K 31/42, A61K 31/50, A61K 31/195, A61K 31/44

(84) Designated Contracting States:
**AT BE CH CY DE DK ES FI FR GB GR IE IT LI LU MC NL PT SE**
Designated Extension States:
**AL LT LV MK RO SI**

(71) Applicant: **Bayer Aktiengesellschaft**
**51368 Leverkusen (DE)**

(72) Inventors:
- **Dr. Hinz, Volker**
  **50670 Köln (DE)**
- **Dr. Haning,Helmut**
  **42115 Wuppertal (DE)**
- **Dr. Riedl Bernd**
  **42329 Wuppertal (DE)**
- **Dr Henning Rolf**
  **42113 Wuppertal (DE)**
- **Dr. Stolle Andreas**
  **Milford CT 06460 (US)**
- **Dr. Keldenich Jörg**
  **42113 Wuppertal (DE)**
- **Dr. Brück Antje**
  **51375 Leverkusen (DE)**
- **Dr. Schumacher Joachim**
  **42109 wuppertal (DE)**

(54) **Use of substituted 4-biarylbutyric and 5-biarylpentanoic acid derivatives for the treatment of cerebral diseases**

(57) Use of substituted 4-Biarylbutyric and 5-Biarylpentanoic Acid Derivatives for the Treatment of Cerebral Diseases, pharmaceutical compositions containing them, and a process for using them.

The compounds of the invention have the generalized formula

$$(T)_xA\text{-}B\text{-}D\text{-}E\text{-}CO_2H$$

wherein A is an aryl or heteroaryl rings; B is an aryl or heteroaryl ring or a bond; each T is a substituent group; x is 0, 1, or 2; the group D represents >C=O , or

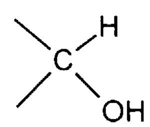
,

the group E represents a two or three carbon chain bearing one to three substituent groups which are independent or are involved in ring formation, possible structures being shown in the text and claims; and each of the substituents on E is an independent substituent; and include pharmaceutically acceptable salts thereof.

EP 1 031 349 A1

## Description

### Field

This invention relates to the use of enzyme inhibitors, and more particularly, to known matrix metalloprotease-inhibiting 4-Biarylbutyric Acids and 5-Biarylpentanoic Acids and derivatives thereof, for the prevention and treatment of cerebral diseases.

### Background

Substituted 4-Biarylbutyric and 5-Biarylpentanoic Acid Derivatives as Matrix Metalloprotease Inhibitors are described in WO 96/15096, WO 97/43237, WO 97/43238, WO 97/43239, WO 97/43240, WO 97/43245, W097/43247 and WO 98/22436.

The matrix metalloproteases (matrix metalloendo-proteinases or MMPs) are a family of zinc endoproteinases which include, but are not limited to, interstitial collagenase (MMP-1), stromelysin (proteoglycanase, transin, or MMP-3), gelatinase A (72kDagelatinase or MMP-2), neutrophil collagenase (MMP-8), gelatinase B (95kDa-gelatinase or MMP-9) and macrophage elastase (MMP-12). These MMPs are secreted by a variety of cells including fibroblasts, chondrocytes, granulocytes and macrophages along with natural proteinatious inhibitors known as TIMPs (Tissue Inhibitor of MetalloProteinase).

All of these MMPs are capable of destroying a variety of connective tissue components of articular cartilage or basement membranes and a wide variety of extracellular matrix proteins. Each MMP is secreted as an inactive proenzyme which must be cleaved in a subsequent step before it is able to exert its own proteolytic activity. In addition to the matrix destroying effect, certain of these MMPs such as MMP-3 have been implemented as the *in vivo* activator for other MMPs such as MMP-1 and MMP-9 (A. Ho, H. Nagase, Arch. Biochem. Biophys., 267, 211-16 (1988); Y. Ogata, J.J. Enghild, H. Nagase, J. Biol. Chem., 267, 3581-84 (1992)). Thus, a cascade of proteolytic activity can be initiated by an excess of MMP-3. It follows that specific MMP-3 inhibitors should limit the activity of other MMPs that are not directly inhibited by such inhibitors.

MMP inhibitors may also be useful in the inhibition of other mammalian metalloproteases such as the adamalysin family (or ADAMs) whose members include TNFα converting enzyme (TACE) and ADAM-10, which can cause the release of TNF from cells.

### Summary

This invention relates to the use for the prevention and treatment of cerebral diseases of compounds having matrix metallprotease inhibitory activity of the generalized formula (I) :

$$(T)_X A\text{-}B\text{-}D\text{-}E\text{-}CO_2H \ . \qquad (I)$$

In the above generalized formula (I), $(T)_X A$ represents a substituted or unsubstituted aromatic 6-membered ring or heteroaromatic 5 - 6 membered ring containing 1 - 2 atoms of N, O, or S. T represents one or more substituent groups, the subscript x represents the number of such substituent groups, and A represents the aromatic or heteroaromatic ring, designated as the A ring or A unit. When N is employed in conjunction with either S or O in the A ring, these heteroatoms are separated by at least one carbon atom.

The substituent group(s) T are independently selected from the group consisting of halogen; alkyl; haloalkyl; haloalkoxy; alkenyl; alkynyl $-(CH_2)_pQ$ in which p is 0 or an integer of 1 - 4; -alkenyl-Q in which the alkenyl moiety comprises 2 - 4 carbons; and alkynyl-Q in which the alkynyl moiety comprises 2 - 7 carbons. Q in the latter three groups is selected from the group consisting of aryl, heteroaryl, -CN, -CHO, $-NO_2$, $-CO_2R^2$, $-OCOR^2$, $-SOR^3$, $-SO_2R^3$, $-CON(R^4)_2$, $-SO_2N(R^4)_2$, $-COR^2$, $-N(R^4)_2$, $-N(R^2)COR^2$, $-N(R^2)CO_2R^3$, $-N(R^2)CON(R^4)_2$, $-CHN_4$, $-OR^4$, and $-SR^4$.

In these formulae $R^2$ represents H, alkyl, aryl, heteroaryl, arylalkyl, or heteroaryl-alkyl. $R^3$ represents alkyl, aryl, heteroaryl, arylalkyl, or heteroaryl-alkyl. $R^4$ represents H; alkyl; aryl; heteroaryl; arylalkyl; heteroaryl-alkyl; alkenyl; alkynyl; alkyleneoxy, polyalkyleneoxy, alkylenethio or alkyleneamino terminated with H, alkyl, or phenyl; haloalkyl; lower alkoxycarbonyl; or acyl. When two $R^4$ groups are situated on a nitrogen, they may be joined by a bond to form a heterocycle, such as, for example, a morpholine, thiomorpholine, pyrrolidine, or piperidine ring.

Unsaturation in a moiety which is attached to Q or which is part of Q is separated from any N, O, or S of Q by at least one carbon atom. The A ring may be unsubstituted or may carry up to 2 substituents T. Accordingly, the subscript x is 0, 1, or 2.

In the generalized formula (I), B represents a bond or an optionally substituted aromatic 6-membered ring or a heteroaromatic 5 - 6 membered ring containing 1 - 2 atoms of N, O, or S. When B is a ring, it is referred to as the B ring or B unit. When N is employed in conjunction with either S or O in the B ring, these heteroatoms are separated by at least one carbon atom. There may be 0 -2 substituents T on ring B.

In the generalized formula (I), D represents >C=O , or

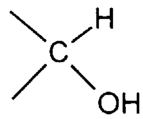

,

in which $R^2$ is defined as above and each $R^2$ may be the same or different.

In the generalized formula (I), E represents a chain of n carbon atoms bearing m substituents $R^6$, in which the $R^6$ groups are independent substituents, or constitute spiro or nonspiro rings. Rings may be formed in two ways: a) two groups $R^6$ are joined, and taken together with the chain atom(s) to which the two R6 group(s) are attached, and any intervening chain atoms, constitute a 3 - 7 membered ring, or b) one group $R^6$ is joined to the chain on which this one group $R^6$ resides, and taken together with the chain atom(s) to which the $R^6$ group is attached, and any intervening chain atoms, constitutes a 3 - 7 membered ring. The number n of carbon atoms in the chain is 2 to 4, and the number m of $R^6$ substituents is an integer of 1 - 3.

Each group $R^6$ is independently selected from the group consisting of:

* fluorine;
* hydroxyl, with the proviso that a single carbon atom may bear no more than one hydroxyl group;
* alkyl;
* aryl;
* heteroaryl;
* arylalkyl;
* heteroaryl-alkyl;
* alkenyl;
* aryl-substituted alkenyl;
* heterarylsubstituted alkenyl;
* alkynyl;
* aryl-substituted alkynyl;
* heteroaryl-substituted alkynyl;
* $-(CH_2)_tR^7$, wherein t is 0 or an integer of 1 - 5 and
  $R^7$ is selected from the group consisting of:

  * N-phthalimidoyl;
  * N-(1,2-naphthalenedicarboximidoyl);
  * N-(2,3-naphthalenedicarboximidoyl);
  * N-(1,8-naphthalenedicarboximidoyl);
  * N-indoloyl;
  * N-(2-pyrrolodinonyl);
  * N-succinimidoyl;
  * N-maleimidoyl;
  * 3-hydantoinyl;
  * 1,2,4-urazolyl;

- * amido;
- * urethane;
- * urea;
- * nonaromatic substituted or unsubstituted heterocycles containing and connected through a N atom, and comprising one or two additional N, O, S, SO, or $SO_2$, and containing zero, one or two carbonyls, and optionally bearing a fused benzene or pyridine ring;
- * amino;
- * corresponding heteroaryl moieties in which the aryl portion of an aryl-containing $R^7$ group comprises 4 - 9 carbons and at least one N, O, or S heteroatom;

* $-(CH_2)_vZR^8$ in which v is 0 or an integer of 1 - 4, wherein
Z represents

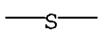 , 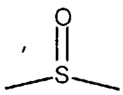 , 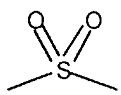 , 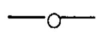 , 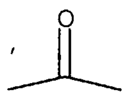 , or 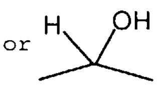 ;

and
$R^8$ is selected from the group consisting of:

- * alkyl;
- * aryl;
- * heteroaryl;
- * arylalkyl;
- * heteroaryl-alkyl; and
- * $-C(O)R^9$ in which $R^9$ represents alkyl of at least two carbons, aryl, heteroaryl, arylalkyl, or heteroaryl-alkyl;

and with the further provisos that

- when $R^8$ is $-C(O)R^9$ , Z is S or O;
- when Z is O, $R^8$ may also be alkyleneoxy or polyalkyleneoxy terminated with H, alkyl, or phenyl; and

* trialkylsilyl-substituted alkyl.

Furthermore, aryl or heteroaryl portions of any of the T or $R^6$ groups optionally may bear up to two substituents selected from the group consisting of $-(CH_2)_yC(R^4)(R^3)OH$, $-(CH_2)_yOR^4$, $-(CH_2)_ySR^4$, $-(CH_2)_yS(O)R^4$, $-(CH_2)_yS(O)_2R^4$, $-(CH_2)_ySO_2N(R^4)_2$, $-(CH_2)_yN(R^4)_2$, $-(CH_2)_yN(R^4)COR^{12}$, $-OC(R^4)_2O-$ in which both oxygen atoms are connected to the aryl ring, $(CH_2)_yCOR^4$, $-(CH_2)_yCON(R^4)_2$, $-(CH_2)_yCO_2R^4$, $-(CH_2)_yOCOR^4$, -halogen, -CHO, $-CF_3$, $-NO_2$, -CN, and $-R^3$, in which y is 0 - 4. $R^3$ and $R^4$ are defined as above; in addition, any two $R^4$ which are attached to one nitrogen may be joined to form a heterocycle such as morpholine, thiomorpholine, pyrrolidine, or a piperidine ring.

Pharmaceutically acceptable salts of these compounds as well as commonly used prodrugs of these compounds such as O-acyl derivatives of invention compounds which contain hydroxy groups are also within the scope of the invention.

In most related reference compounds of the prior art, the biphenyl portion of the molecule is unsubstituted, and the propanoic or butanoic acid portion is either unsubstituted or has a single methyl or phenyl group. Presence of the larger phenyl group has been reported to cause prior art compounds to be inactive as anti-inflammatory analgesic agents. See, for example, R.G. Child, et al., J. Pharm. Sci., 66, 466-476 (1977). By contrast, it has now been found that compounds which exhibit potent MMP inhibitory activity contain a substituent of significant size on the propanoic or butanoic portion of the molecule. The biphenyl portions of the best MMP inhibitors also preferably contain a substituent on the 4' position, although when the propanoic or butanoic portions are optimally substituted, the unsubstituted biphenyl compounds of the invention have sufficient activity to be considered realistic drug candidates.

In addition to the above-described compounds, the invention also relates to pharmaceutical compositions having matrix metalloprotease inhibitory activity, which compositions comprise a compound of the invention as described above and in more detail in the detailed description below, and a pharmaceutically acceptable carrier.

The invention also relates to a method of treating a mammal such as a human, a farm animal, or a domestic pet, to achieve an effect, in which the effect is: treatment and prevention of acute and chronic neurodegenerative disorders including stroke, spinal cord and traumatic brain injury, amyotrophic lateral sclerosis, cerebral amyloid angiopathy, CNS injuries in AIDS, Parkinson's disease, Alzheimer's disease, Huntington's diseases, prion diseases, myasthenia gravis, Duchenne's muscular dystrophy; diseases linked to $TNF_{\alpha}$ production including myelodysplastic syndromes, acute encephalitis; pain; neuropathies including drug induced neuropathy, mechanical damage neuropathy; intoxications including drug induced intoxication, alcohol induced intoxication; migraine; aneurysmal diseases including those of the brain; brain edemas as a result of pre-operative treatment for brain and spinal surgery including cerebral blood vessel surgery, removal of surface tumors; pre-operative treatment during cardiac by-pass surgery; depression; schizophrenia; anxiety the method comprising administering an amount of a compound of the invention as described above, and in more detail in the detailed description below, which is effective to inhibit the activity of at least one matrix metalloprotease, resulting in achievement of the desired effect.

**Detailed Description**

More particularly preferred are for the use for the prevention and treatment of cerebral diseases are compounds having matrix metalloprotease inhibitory activity of the generalized formula:

$$(T)_X A\text{-}B\text{-}D\text{-}E\text{-}CO_2H \qquad (I)$$

in which $(T)_X A$ represents a substituted or unsubstituted aromatic or heteroaromatic moiety selected from the group consisting of:

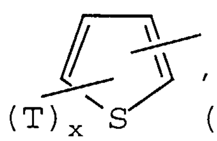 , 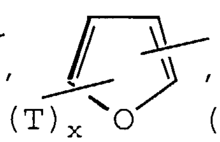 , 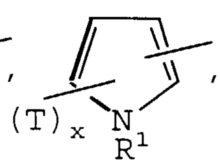 , 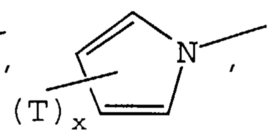 ,

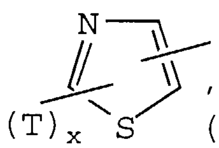 , 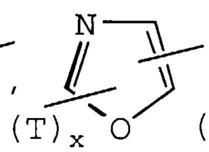 , 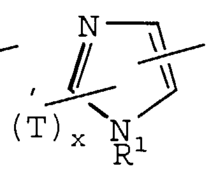 , 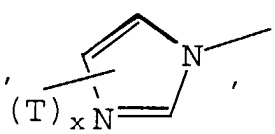 ,

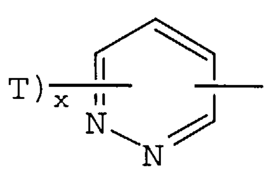 , 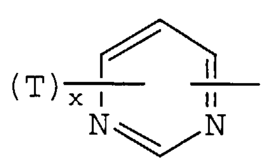 , 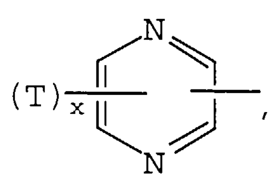 ,

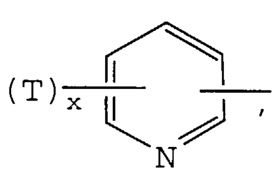 , and 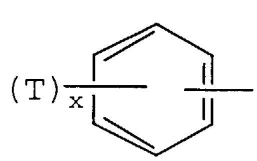

in which $R^1$ represents H or alkyl of 1 - 3 carbons.

In these structures, the aromatic ring is referred to as the A ring or A unit, and each T represents a substituent group, referred to as a T group or T unit. Substituent groups T are independently selected from the group consisting of: the halogens -F, -Cl, -Br, and -I; alkyl of 1 - 10 carbons; haloalkyl of 1 - 10 carbons; haloalkoxy of 1 - 10 carbons; alkenyl of 2 - 10 carbons; alkynyl of 2 - 10 carbons; $-(CH_2)_pQ$ in which p is 0 or an integer 1 - 4; -alkenyl-Q in which the alkenyl moiety comprises 2 - 4 carbons; and -alkynyl-Q in which the alkenyl moiety comprises 2 - 7 carbons. Q in each of the latter three groups is selected from the group consisting of aryl of 6 - 10 carbons; heteroaryl comprising 4 - 9 carbons and at least one N, O, or S heteroatom; -CN; -CHO; $-NO_2$; $-CO_2R^2$; $-OCOR^2$; $-SOR^3$; $-SO_2R^3$; $-CON(R^4)_2$ ; $-SO_2N(R^4)_2$ ; $-C(O)R^2$ ; $-N(R^4)_2$; $-N(R^2)COR^2$; $-N(R^2)CO_2R^3$; $-N(R^2)CON(R^4)_2$; $-CHN_4$; $-OR^4$; and $-SR^4$. The groups $R^2$, $R^3$, and $R^4$ are defined as follows.

$R^2$ represents H; alkyl of 1 - 6 carbons; aryl of 6 - 10 carbons; heteroaryl comprising 4 - 9 carbons and at least one N, O, or S heteroatom; arylalkyl in which the aryl portion contains 6 - 10 carbons and the alkyl portion contains 1 - 4 carbons; or heteroaryl-alkyl in which the heteroaryl portion comprises 4 - 9 carbons and at least one N, O, or S heteroatom and the alkyl portion contains 1 - 4 carbons.

$R^3$ represents alkyl of 1 - 4 carbons; aryl of 6 - 10 carbons; heteroaryl comprising 4 - 9 carbons and at least one N, O, or S heteroatom; arylalkyl in which the aryl portion contains 6 - 10 carbons and the alkyl portion contains 1 - 4 carbons; or heteroaryl-alkyl in which the heteroaryl portion comprises 4 - 9 carbons and at least one N, O, or S heteroatom and the alkyl portion contains 1 - 4 carbons.

$R^4$ represents H; alkyl of 1 - 12 carbons; aryl of 6 - 10 carbons; heteroaryl comprising 4 - 9 carbons and at least one N, O, or S heteroatom; arylalkyl in which the aryl portion contains 6 - 10 carbons and the alkyl portion contains 1 - 4 carbons; heteroaryl-alkyl in which the heteroaryl portion comprises 4 - 9 carbons and at least one N, O, or S heteroatom and the alkyl portion contains 1 - 4 carbons; alkenyl of 2 - 12 carbons; alkynyl of 2 - 12 carbons; $-(C_qH_{2q}O)_rR^5$ in which q is 1-3, r is 1 - 3, and $R^5$ is H provided q is greater than 1, or $R^5$ is alkyl of 1 - 4 carbons, or phenyl; alkylenethio terminated with H, alkyl of 1-4 carbons, or phenyl; alkyleneamino terminated with H, alkyl of 1-4 carbons, or phenyl; $-(CH_2)_sX$ in which s is 1-3 and X is halogen; $-C(O)OR^2$; or $-C(O)R^2$.

When two $R^4$ groups are situated on a nitrogen, they may be joined by a bond to form a heterocycle, such as, for example, a morpholine, thiomorpholine, pyrrolidine, or piperidine ring.

Any unsaturation in a moiety which is attached to Q or which is part of Q is separated from any N, O, or S of Q by at least one carbon atom, and the number of substituents, designated x, is 0, 1, or 2.

In the generalized formula (I), B represents a bond or an optionally substituted aromatic or heteroaromatic ring selected from the group consisting of:

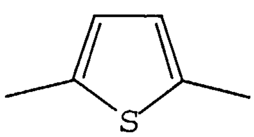 , 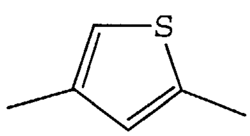 , 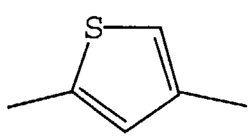 , 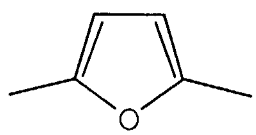 ,

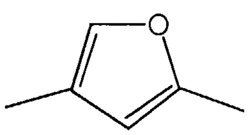 , 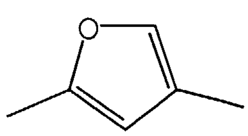 , 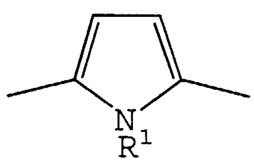 , 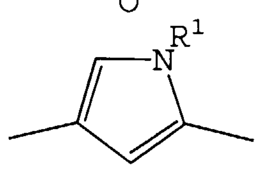 ,

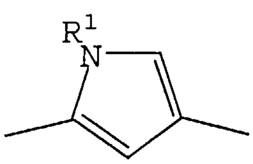 , 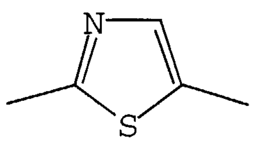 , 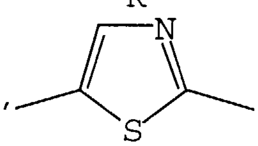 , 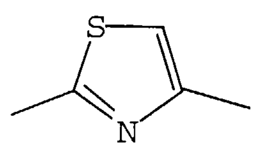 ,

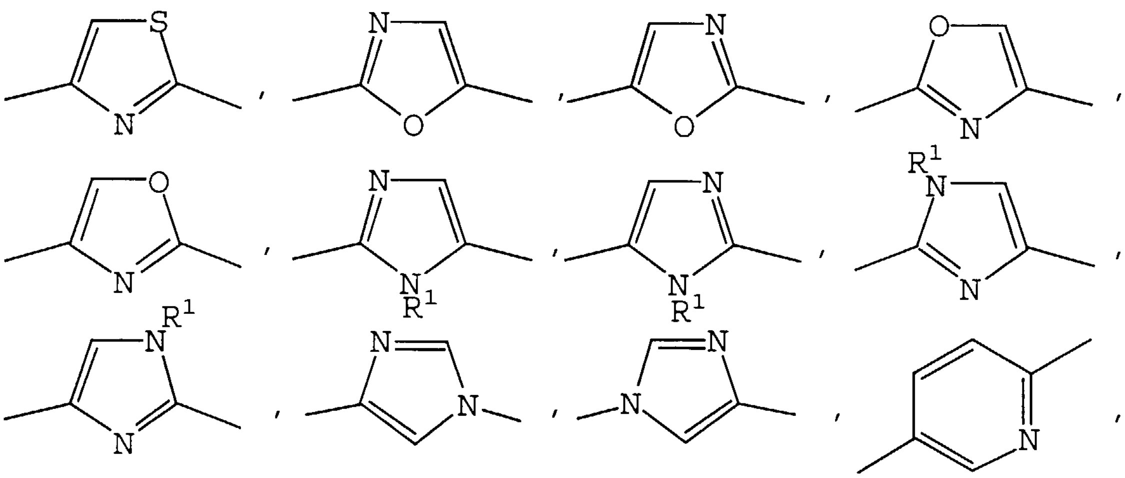

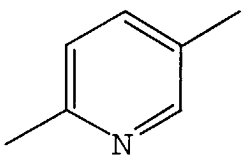 , 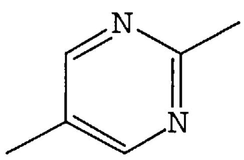 , 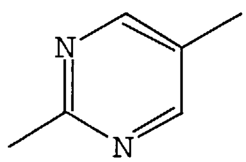 , 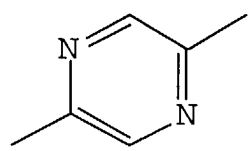 ,

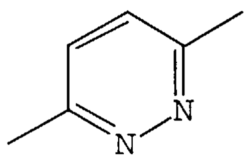 , and 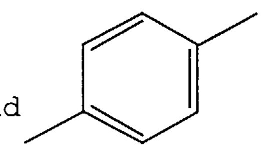

in which $R^1$ is defined as above and each $R^1$ may be the same or different. These rings are referred to as the B ring or B unit. There may be 0-2 substituents T on the B ring, T being defined as above.

In the generalized formula (I), D represents the moieties $\rangle$C=O , or

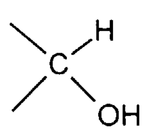 ,

In the generalized formula (I), E represents a chain of n carbon atoms bearing m substituents $R^6$, referred to as $R^6$ groups or $R^6$ units. The $R^6$ groups are independent substituents, or constitute spiro or nonspiro rings. Rings may be formed in two ways: a) two groups $R^6$ are joined, and taken together with the chain atom(s) to which the two R6 group(s) are attached, and any intervening chain atoms, constitute a 3 - 7 membered ring, or b) one group $R^6$ is joined to the chain on which this one group $R^6$ resides, and taken together with the chain atom(s) to which the $R^6$ group is attached, and any intervening chain atoms, constitutes a 3 - 7 membered ring. The number n of carbon atoms in the chain is 2 or 3, and the number m of $R^6$ substituents is an integer of 1 - 3.

Each group $R^6$ is independently selected from the group consisting of the substituents listed below as items 1) - 16).

1) fluorine;

2) hydroxyl, with the proviso that a single carbon atom may bear no more than one hydroxyl group;

3) alkyl of 1 - 10 carbons;

4) aryl of 6- 10 carbons;

5) heteroaryl comprising 4 - 9 carbons and at least one N, O, or S heteroatom;

6) arylalkyl in which the aryl portion contains 6 - 10 carbons and the alkyl portion contains 1 - 8 carbons;

7) heteroaryl-alkyl in which the heteroaryl portion comprises 4 - 9 carbons and at least one N, O, or S heteroatom, and the alkyl portion contains 1 - 8 carbons;

8) alkenyl of 2- 10 carbons;

9) aryl-alkenyl in which the aryl portion contains 6 - 10 carbons and the alkenyl portion contains 2 - 5 carbons;

10) heteroaryl-alkenyl in which the heteroaryl portion comprises 4 - 9 carbons and at least one N, O, or S heteroatom and the alkenyl portion contains 2 -5 carbons;

11) alkynyl of 2- 10 carbons;

12) aryl-alkynyl in which the aryl portion contains 6 - 10 carbons and the alkynyl portion contains 2 - 5 carbons;

13) heteroaryl-alkynyl in which the heteroaryl portion comprises 4 - 9 carbons and at least one N, O, or S heteroatom and the alkynyl portion contains 2 - 5 carbons;

14) $-(CH_2)_tR^7$ in which t is 0 or an integer of 1 - 5 and $R^7$ is selected from the group consisting of

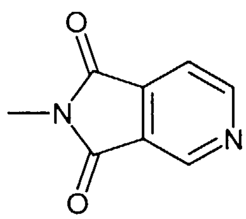
,

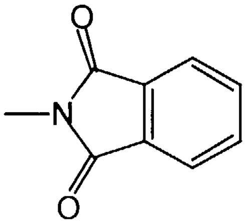 , 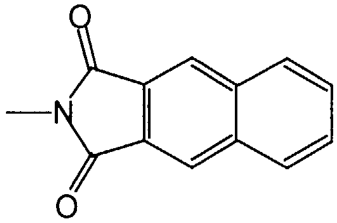 , 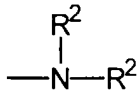 , 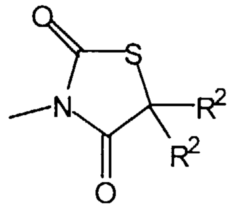 ,

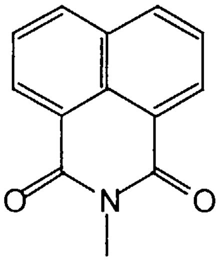 , 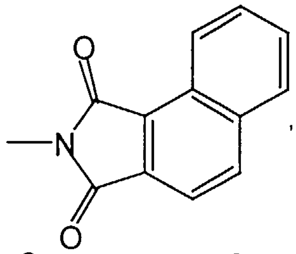 , 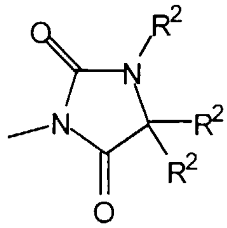 , 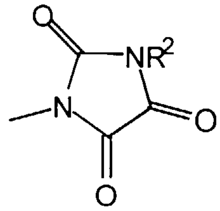 ,

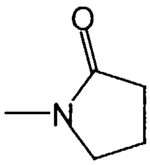 , 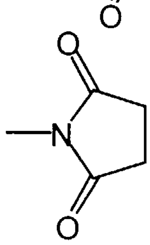 , 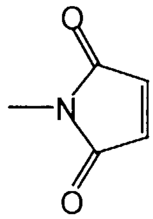 , 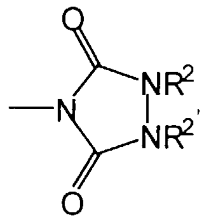 , 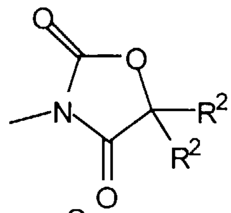 ,

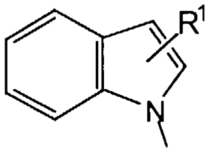 , 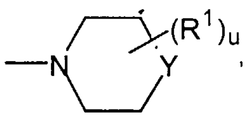 , 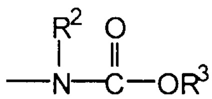 , 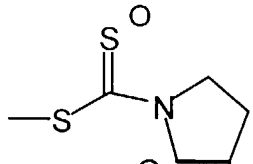 ,

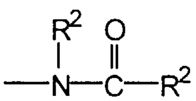 , 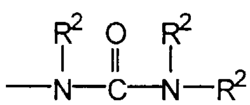 , 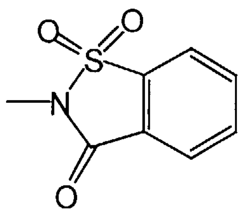 , 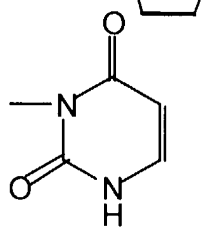 ,

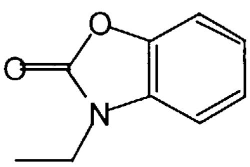 , 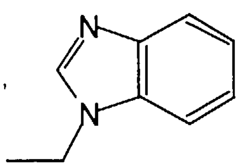 , 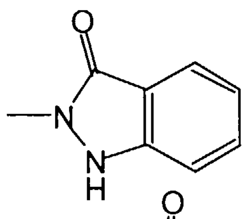 , 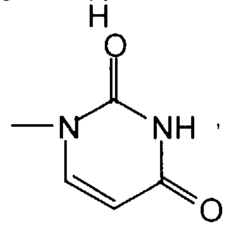 ,

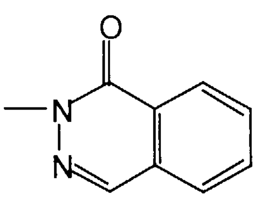 , 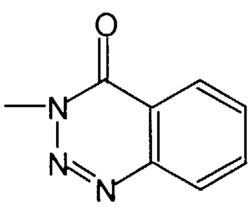 , 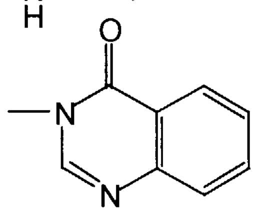 ,

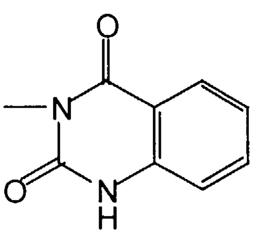 , 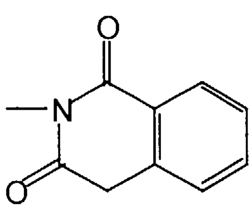 , 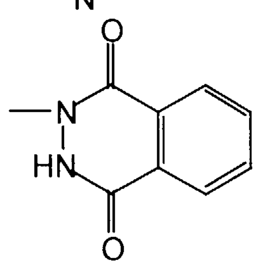 ,

as well as corresponding heteroaryl moieties in which the aryl portion of an aryl-containing $R^7$ group comprises 4

- 9 carbons and at least one N, O, or S heteroatom. In such $R^7$ groups, Y represents O or S; $R^1$, $R^2$, and $R^3$ are as defined above, and each $R^1$, $R^2$ and $R^3$ may be the same or different; and u is 0, 1, or 2;

15) $-(CH_2)_vZR^8$ in which v is 0 or an integer of 1 to 4; Z represents -S-, -S(O)-, $-SO_2-$, -O-, carbonyl, or -CH(OH)-; and $R^8$ is selected from the group consisting of: alkyl of 1 to 12 carbons; aryl of 6 to 10 carbons; heteroaryl comprising 4 - 9 carbons and at least one N, O, or S heteroatom; arylalkyl in which the aryl portion contains 6 to 10 carbons and the alkyl portion contains 1 to 4 carbons; heteroaryl-alkyl in which the aryl portion comprises 4 - 9 carbons and at least one N, O, or S heteroatom and the alkyl portion contains 1 - 4 carbons; $-C(O)R^9$ in which $R^9$ represents alkyl of 2 - 6 carbons, aryl of 6 - 10 carbons, heteroaryl comprising 4 - 9 carbons and at least one N, O, or S heteroatom, or arylalkyl in which the aryl portion contains 6 - 10 carbons or is heteroaryl comprising 4 - 9 carbons and at least one N, O, or S heteroatom, and the alkyl portion contains 1 - 4 carbons, with the provisos that

- when $R^8$ is $-C(O)R^9$ , Z is -S- or -O-;
- when Z is -O-, $R^8$ may also be $-(C_qH_{2q}O)_rR^5$ in which q, r, and $R^5$ are as defined above;

16) $-(CH_2)_wSi(R^{10})_3$ in which w is an integer of 1 to 3, and $R^{10}$ represents alkyl of 1 to 4 carbons.

In addition, aryl or heteroaryl portions of any of the T or $R^6$ groups optionally may bear up to two substituents selected from the group consisting of $-(CH_2)_yC(R^4)(R^3)OH$, $-(CH_2)_yOR^4)$, $-(CH_2)_ySR^4)$, $-(CH_2)_yS(O)R^4)$, $-(CH_2)_yS(O)_2R^4)$, $-(CH_2)_ySO_2N(R^4))_2$, $-(CH_2)_yN(R^4))_2$, $-(CH_2)_yN(R^4))COR^3$, $-OC(R^4))_2O-$ in which both oxygen atoms are connected to the aryl ring, $-(CH_2)_yCOR^4$, $-(CH_2)_yCON(R^4))_2$, $-(CH_2)_yCO_2R^4)$, $-(CH_2)_yOCOR^4)$, -halogen, -CHO, $-CF_3$, $-NO_2$, -CN, and $-R^3$, in which y is 0 - 4; $R^3$ is defined as above; $R^4$ is defined as above, and each $R^3$ or $R^4$ may be the same or different and in addition, any two $R^4$ which are attached to one nitrogen may be joined to form a heterocycle, such as a morpholine, thiomorpholine, pyrrolidine, or piperidine ring.

Pharmaceutically acceptable salts of these compounds as well as commonly used prodrugs of these compounds such as O-acyl derivatives of these compounds are also within the scope of the invention.

In the compounds of the invention, the following are preferred.

The substituent group T, when it is on the ring A, is preferably halogen, 1-alkynyl-Q, or an ether $OR^4$ wherein $R^4$ is preferably alkyl of 1 - 12 carbons or arylalkyl in which the aryl portion is 6 - 10 carbons and the alkyl portion contains 1 - 4 carbons. Most preferably, T is halogen, or $-C\equiv C-(CH_2)_tOH$ in which t is an integer of 1 - 5, and when T is $OR^4$, $R^4$ is alkyl of 1 - 6 carbons, or benzyl.

The subscript x, which defines the number of T substituents, is preferably 1 or 2, most preferably 1, and this substituent T is preferably on the 4- position of ring A.

The A ring is preferably a phenyl or thiophene ring, most preferably phenyl. The A ring preferably bears at least one substituent group T, preferably located on the position furthest from the position of the A ring which is connected to the B ring.

The B moiety of generalized formula (I) is a bond or a substituted or unsubstituted aromatic or heteroaromatic ring, in which any substituents are groups which do not cause the molecule to fail to fit the active site of the target enzyme, or disrupt the relative conformations of the A and B rings, such that they would be detrimental. Such groups may be, but are not limited to, moieties such as lower alkyl, lower alkoxy, CN, $NO_2$, halogen, etc. The B moiety is preferably a 1,4-phenylene or 2,5-thiophene ring, most preferably 1,4-phenylene.

The D unit is most preferably a carbonyl or a -CHOH- group.

The group $R^6$ is preferably:

1) arylalkyl wherein the aryl portion contains 6 - 10 carbons and the alkyl portion contains 1 - 8 carbons;

2) $-(CH_2)_tR^7$ wherein t is 0 or an integer of 1 - 5 and $R^7$ is an imidoyl group fused to an aromatic residue, or the 1,2,3-benzotriazin-4(3H)-one-3-yl group; or

3) $-(CH_2)_vZR^8$ wherein v is 0 or an integer of 1 - 4 , Z is S or O, and $R^8$ is aryl of 6 - 10 carbons or arylalkyl wherein the aryl portion contains 6 to 12 carbons and the alkyl portion contains 1 to 4 carbons.

The group $R^6$ is most preferably one of the following, and in these, any aromatic moiety is preferably substituted:

1) arylalkyl wherein the aryl portion is phenyl and the alkyl portion contains 1 - 4 carbons;

2) $-(CH_2)_tR^7$ wherein t is an integer of 1 - 3, and $R^7$ is N-phthalimidoyl, 1,2,3-benzotriazin-4(3*H*)-one-3-yl, N-(1,2-naphthalenedicarboximidoyl), N-(2,3-naphthalenedicarboximidoyl), or N-(1,8-naphthalenedicarboximidoyl); or

3) $-(CH_2)_vZR^8$ wherein v is an integer of 1 - 3, Z is S, and $R^8$ is phenyl.

It is to be understood that as used herein, the term "alkyl" means straight, branched, cyclic, and polycyclic materials. The term "haloalkyl" means partially or fully halogenated alkyl groups such as $-(CH_2)_2Cl$, $-CF_3$ and $-C_6F_{13}$, for example.

In one of its embodiments, the invention relates to compounds of generalized formula (I) in which at least one of the units A, B, T, and $R^6$ comprises a heteroaromatic ring. Preferred heteroaromatic ring-containing compounds are those in which the heteroaryl groups are heteroaryl of 4 - 9 carbons comprising a 5 - 6 membered heteroaromatic ring containing O, S, or $NR^1$ when the ring is 5-membered, and N when said ring is 6-membered. Particularly preferred heteroaromatic ring-containing compounds are those in which at least one of the A and B units comprises a thiophene ring. When A unit is thiophene, it is preferably connected to B unit at position 2 and carries one substituent group T on position 5. When B Unit is thiophene, it is preferably connected through positions 2 and 5 to D and A units respectively.

In another embodiment, the invention relates to compounds of generalized formula (I), in the E unit of which n is 2 and m is 1. These compounds thus possess two carbon atoms between the D unit and carboxyl group, and carry one substituent on this two-carbon chain.

In another of its embodiments, the invention relates to compounds of generalized formula (I) in which the A ring is a substituted or unsubstituted phenyl group, the B ring is p-phenylene, and aryl portions of any aryl-containing T and $R^6$ moieties contain only carbon in the rings. These compounds thus contain no heteroaromatic rings.

In another of its embodiments, the invention relates to compounds of generalized formula (I) in which m is 1 and $R^6$ is an independent substituent. These compounds are materials which contain only a single substituent $R^6$ on the E unit, and this substituent in not involved in a ring.

Preferred compounds of general formula (I) in which $R^6$ is $-(CH_2)_tR^7$ have t as an integer of 1-5. Preferred compounds of general formula (I) in which $R^6$ is $-(CH_2)_vZR^8$ have v as an integer of 1-4 and Z as -S- or -O-. Preferred compounds of general formula (I) in which $R^6$ is alkyl contain 4 or more carbons in said alkyl and those in which $R^6$ is arylalkyl contain 2-3 carbons in the alkyl portion of said arylalkyl.

In another of its embodiments, the invention relates to compounds of generalized formula (I) in which the number of substituents m on the E unit is 2 or 3; and when m is 2, both groups $R^6$ are independent substituents, or together constitute a spiro ring, or one group $R^6$ is an independent substituent and the other constitutes a spiro ring; and when m is 3, two groups $R^6$ are independent substituents and one group $R^6$ constitutes a ring, or two groups $R^6$ constitute a ring and one group $R^6$ is an independent substituent, or three groups R6 are independent substituents. This subset therefore contains compounds in which the E unit is di- or trisubstituted, and in the disubstituted case any rings formed by one or both $R^6$ groups are spiro rings, and in the trisubstituted case, the $R^6$ groups may form either spiro or nonspiro rings.

In another of its embodiments, the invention relates to compounds of generalized formula (I) in which the number of substituents m on the E unit is 1 or 2; and when m is 1, the group $R^6$ constitutes a nonspiro ring; and when m is 2, both groups $R^6$ together constitute a nonspiro ring or one group $R^6$ is an independent substituent and the other constitutes a nonspiro ring. This subset therefore contains compounds in which the E unit carries one or two substituents $R^6$, and at least one of these substituents is involved in a nonspiro ring.

More particularly, representative compounds of generalized formula (I) in which one or more of the substituent groups $R^6$ are involved in formation of nonspiro rings have E units of the following structures:

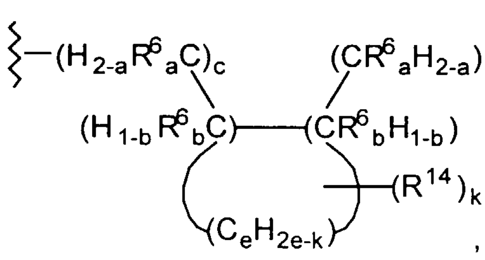
,
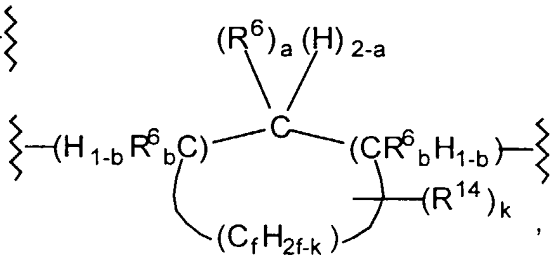
,

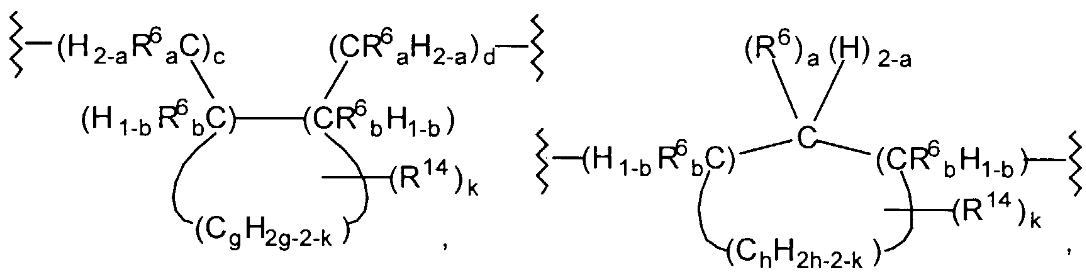

, ,

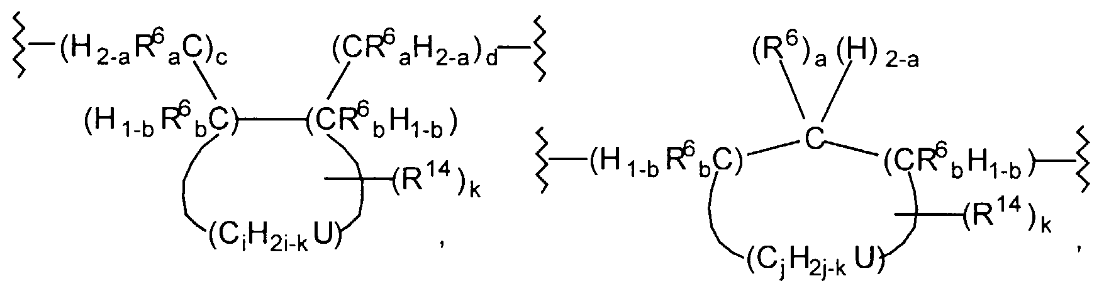

, ,

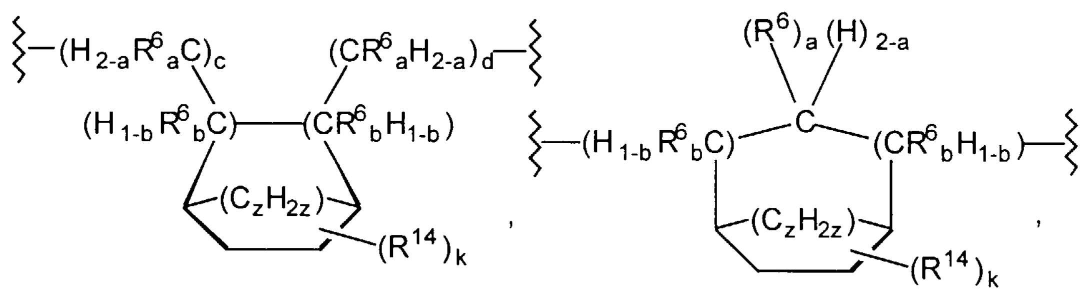

, ,

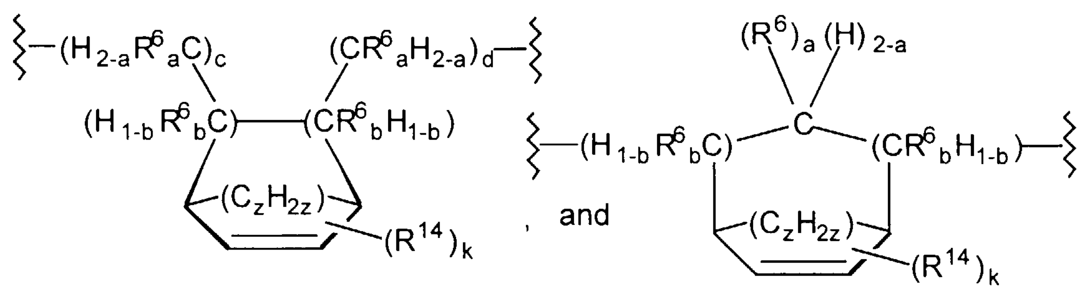

, and

in which a is 0, 1, or 2; b is 0 or 1; c is 0 or 1; d is 0 or 1; c + d is 0 or 1; e is 1 - 5; f is 1 - 4; g is 3 - 5; h is 2 - 4; i is 0 - 4; j is 0 - 3; k is 0 - 2; the total number of groups $R^6$ is 0, 1, or 2; U represents O, S, or $NR^1$; and z is 1 or 2; Each group $R^{14}$ is independently selected from the group consisting of: alkyl of 1 - 9 carbons; arylalkyl in which the alkyl portion contains 1 - 7 carbons and the aryl portion contains 6 - 10 carbons; alkenyl of 2 - 9 carbons; aryl-substituted alkenyl in which the alkenyl portion contains 2 - 4 carbons and the aryl portion contains 6 - 10 carbons; alkynyl of 2 - 9 carbons; aryl-substituted alkynyl in which the alkynyl portion contains 2 - 4 carbons and the aryl portion contains 6 - 10 carbons; aryl of 6 - 10 carbons; $-COR^2$; $-CH(OH)R^2$; $-CO_2R^3$; $-CON(R^2)_2$; $-(CH_2)_tR^7$ in which t is 0 or an integer of 1 - 4; and $-(CH_2)_vZR^8$ in which v is 0 or an integer of 1 to 3, and Z represents -S-, S(O), $SO_2$, or -O-. $R^1$, $R^7$, and $R^8$ have been defined above.

Preferred compounds of generalized formula (I) in which one or more of the substituent groups $R^6$ are involved in formation of nonspiro rings have E units of the following structures:

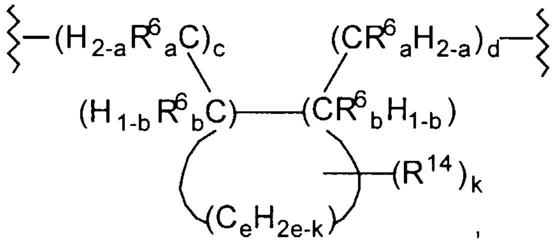
,
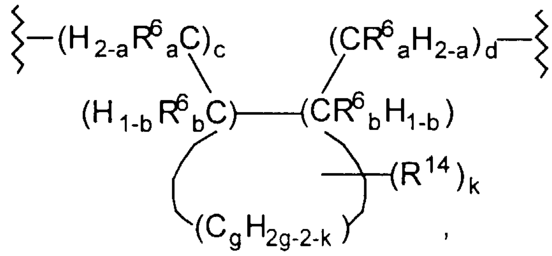
,

and
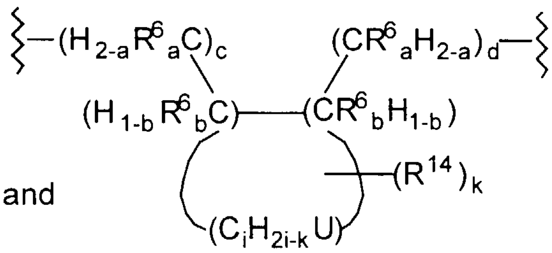

in which a, b, c, d, (c + d), e, g, i, k, the total number of groups $R^6$, U, and $R^{14}$ are as defined above.

The more preferred compounds for the use for the prevention and treatment of respiratory diseases of generalized formula (I), in which one or more of the substituent groups $R^6$ are involved in formation of nonspiro rings have the formula

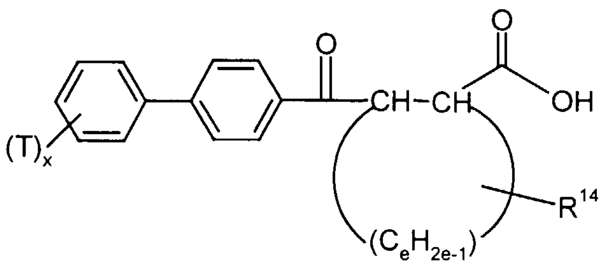

in which the subscript x is 1 or 2; one substituent T is located on the 4-position of the A ring, relative to the point of attachment between the A and B rings; e is 2 or 3; and $R^{14}$ is as defined above.

Very particularly preferred compounds are those shown in table 1:

| Ex. | Structure | Isomer | corresponds to Example | in WO-A- |
|---|---|---|---|---|
| 1 | 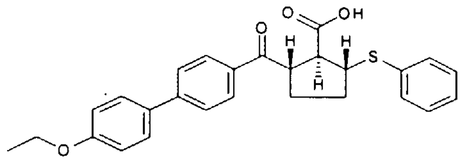 | RACEMATE | **315** | **96/15096** |
| 2 | 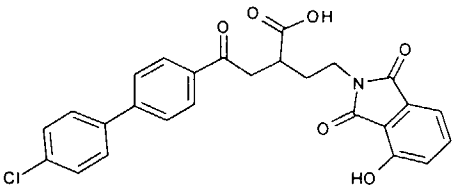 | RACEMATE | **421** | **96/15096** |
| 3 | 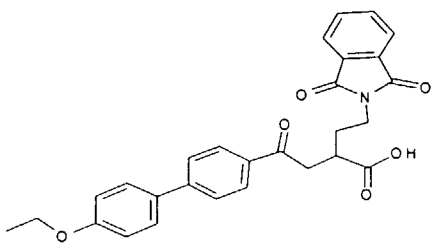 | RACEMATE | **273** | **96/15096** |
| 4 | 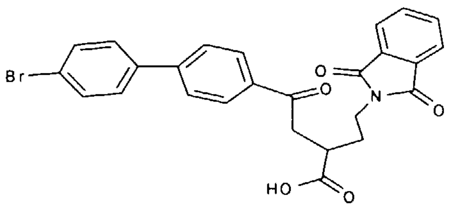 | RACEMATE | **270** | **96/15096** |
| 5 | 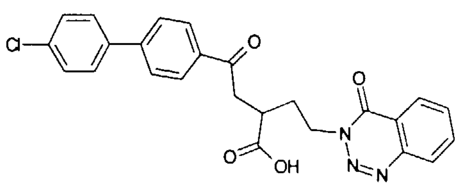 | SECOND ENANTIOMER OFF CHIRAL COLUMN 1 | **21** | **97/43239** |
| 6 | 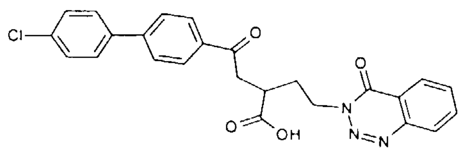 | RACEMATE | **19** | **97/43239** |
| 7 | 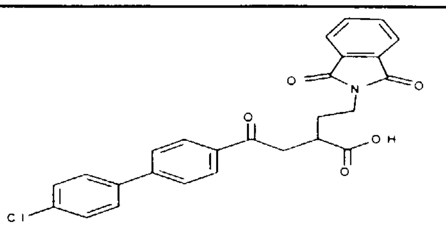 | RACEMATE | **267** | **96/15096** |

| Ex. | Structure | Isomer | corresponds to Example | in WO-A- |
|---|---|---|---|---|
| 8 | 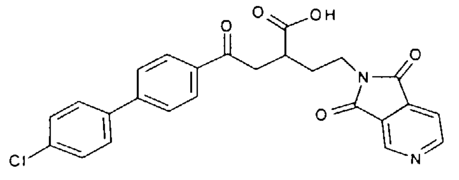 | RACEMATE | **417** | **96/15096** |
| 9 | 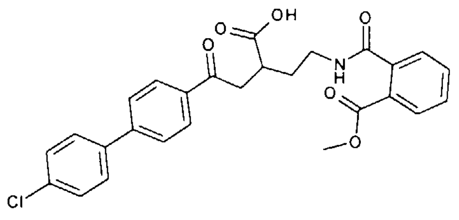 | RACEMATE | **404** | **96/15096** |
| 10 | 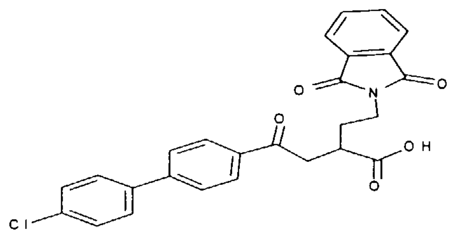 | FIRST ENANTIOMER OFF CHIRAL COLUMN 2 | **268** | **96/15096** |
| 11 | 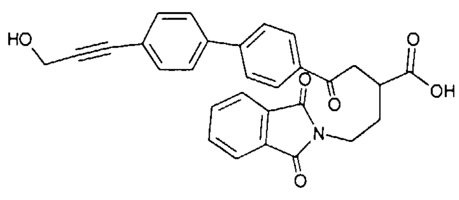 | RACEMATE | **16** | **97/43245** |
| 12 | 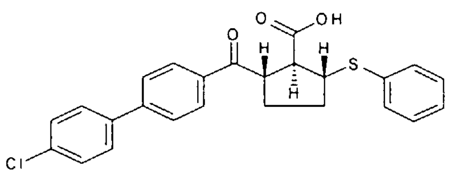 | (+)-ENANTIOMER | **296** | **96/15096** |
| 13 | 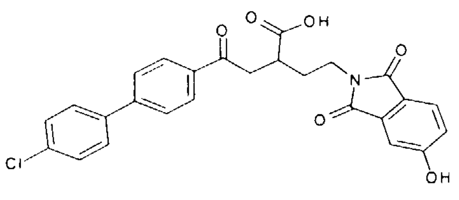 | RACEMATE | **420** | **96/15096** |
| 14 | 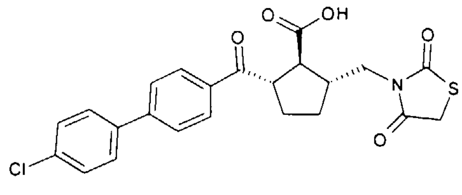 | RACEMATE | **8** | **97/43239** |

| Ex. | Structure | Isomer | corresponds to Example | in WO-A- |
|---|---|---|---|---|
| 15 | 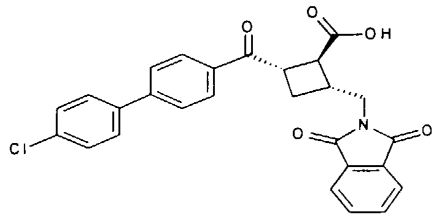 | RACEMATE | **402** | **96/15096** |
| 16 | 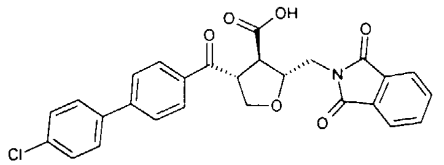 | RACEMATE | **403** | **96/15096** |
| 17 | 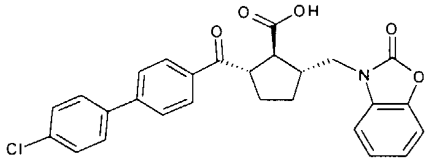 | RACEMATE | **6** | **97/43239** |
| 18 | 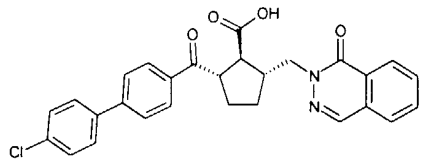 | RACEMATE | **5** | **97/43239** |
| 19 | 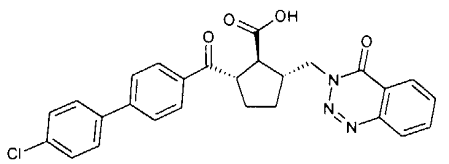 | RACEMATE | **1** | **97/43239** |
| 20 | 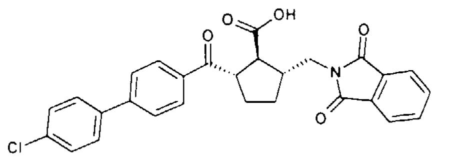 | RACEMATE | **360** | **96/15096** |
| 21 | 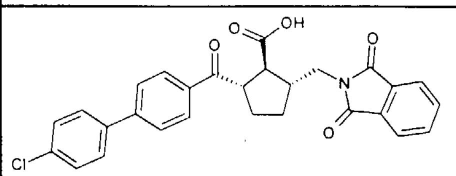 | FIRST ENANTIOMER OFF CHIRAL COLUMN 3 | **361** | **96/15096** |
| 22 | 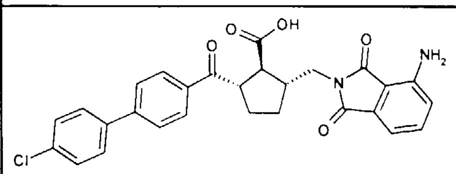 | RACEMATE | **392** | **96/15096** |

| Ex. | Structure | Isomer | corresponds to Example | in WO-A- |
|---|---|---|---|---|
| 23 | 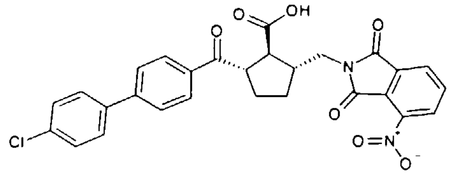 | RACEMATE | **385** | **96/15096** |
| 24 | 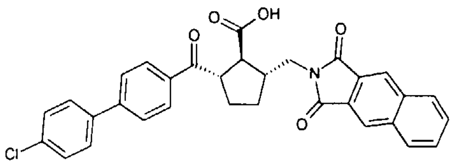 | RACEMATE | **387** | **96/15096** |
| 25 | 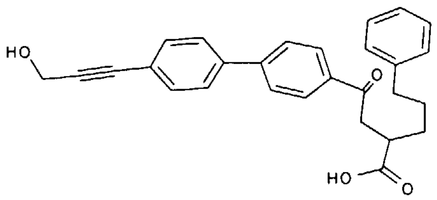 | SECOND ENANTIOMER OFF CHIRAL COLUMN 4 | **3** | **97/43245** |
| 26 | 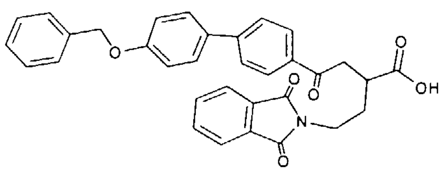 | RACEMATE | **271** | **96/15096** |
| 27 | 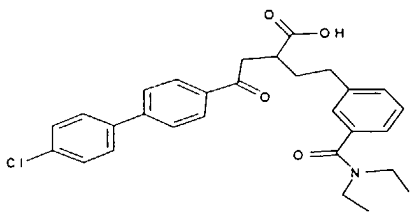 | FIRST ENANTIOMER OFF CHIRAL COLUMN 3 | **145** | **96/15096** |
| 28 | 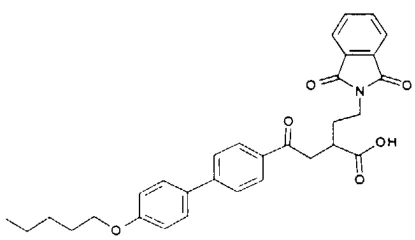 | RACEMATE | **272** | **96/15096** |
| 29 | 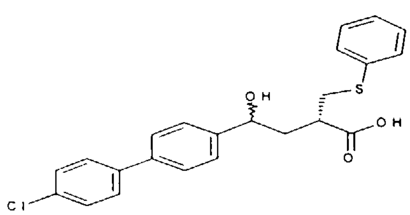 | RACEMATE | **1** | **98/22436** |

| Ex. | Structure | Isomer | corresponds to Example | in WO-A- |
|---|---|---|---|---|
| 30 | 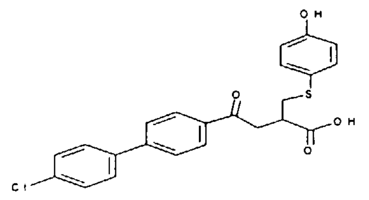 | FIRST ENANTIOMER OFF CHIRAL COLUMN 1 | **204** | **98/22436** |
| 31 | 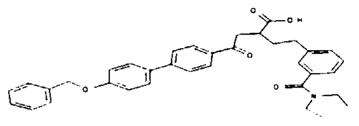 | RACEMATE | **165** | **96/15096** |
| 32 | 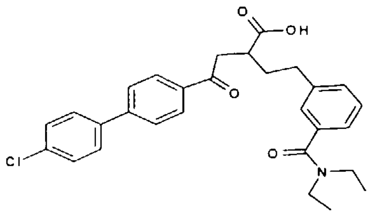 | RACEMATE | **149** | **96/15096** |
| 33 | 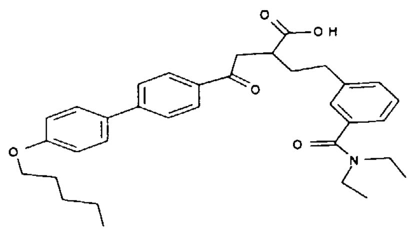 | RACEMATE | **164** | **96/15096** |
| 34 | 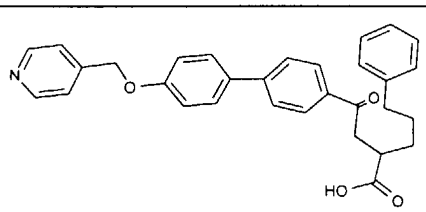 | RACEMATE | **111** | **96/15096** |
| 35 | 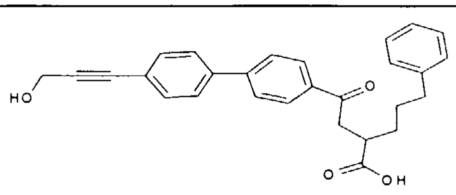 | RACEMATE | **1** | **97/43245** |
| 36 | 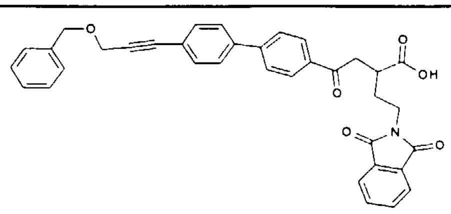 | RACEMATE | **15** | **97/43245** |

| Ex. | Structure | Isomer | corresponds to Example | in WO-A- |
|---|---|---|---|---|
| 37 | 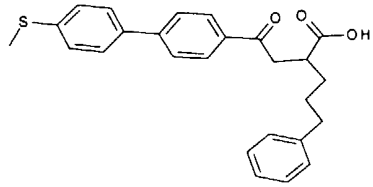 | RACEMATE | 51 | 96/15096 |
| 38 | 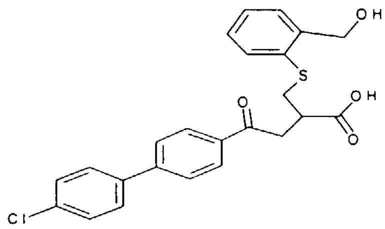 | RACEMATE | 217 | 96/15096 |
| 39 | 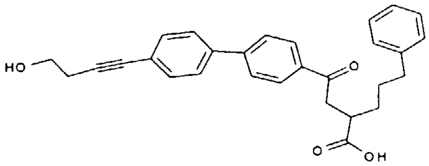 | RACEMATE | 8 | 97/43245 |
| 40 | 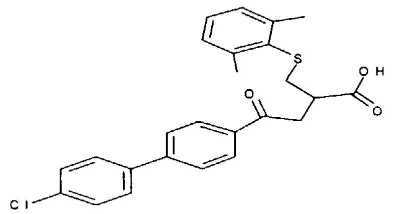 | RACEMATE | 433 | 96/15096 |
| 41 | 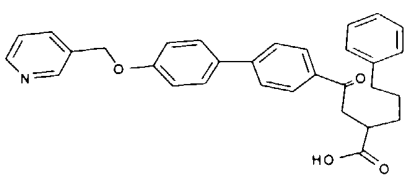 | RACEMATE | 109 | 96/15096 |
| 42 | 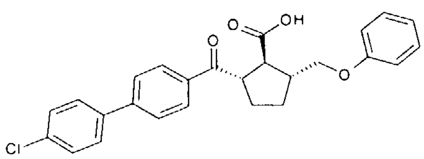 | RACEMATE | 366 | 96/15096 |
| 43 | 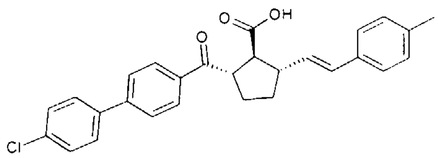 | RACEMATE | 363 | 96/15096 |
| | chiral column 1 = described in Arlt et al. Angew.Chem.Int.Ed.Engl. 1991, 30, 1662-1664 using monomer 2b in Table | | | |

| Ex. | Structure | Isomer | corresponds to Example | in WO-A- |
|---|---|---|---|---|
| | chiral column 2 = Chiralpak AD | | | |
| | chiral column 3 = Chiralpak AS | | | |
| | chiral column 4 = Chiralcel AD | | | |
| | chiral column 5 = analogous to HPLC-columns in Arlt et al. Angew.Chem.Int.Ed.Engl. 1991, 30, 1662-1664 using as monomer N-(methacryloyl)-L-valine-3-pentylamide | | | |

table 1

In the above structures, the term "racemate" in case of the cyclopentane derivatives refers to the trans, trans-diastereomer.

**General Preparative Methods:**

The compounds of the invention may be prepared by use of known chemical reactions and procedures as described in details in WO 96/15096, WO 97/43237, WO 97/43238, WO 97/43239, WO 97/43240, WO 97/43245, W097/43247 and WO 98/22436. Nevertheless, the following general preparative methods are presented to aid the reader in synthesizing the inhibitors. General methods A through K may be used to prepare appropriately substituted 4-biaryl-4-oxobutanoic acids, 4-aryl-4-oxo-butanoic acids, 5-biaryl-5-oxopentanoic acids, or 5-aryl-5-oxopentanoic acids. These general methods are also found in WO 96/15096 along with exemplary preparations of the keto acids. The choice of a specific synthetic method is dictated by the proviso that the conditions used do not effect undesired changes in the T or $R^6$ moieties of the compounds prepared.

All variable groups of these methods are as described in the generic description if they are not specifically defined below. The variable subscript n is independently defined for each method. When a variable group with a given symbol (i.e. $R^6$ or T) is used more than once in a given structure, it is to be understood that each of these groups may be independently varied within the range of definitions for that symbol. As defined above, the compounds of the invention contain as the E unit a chain of 2 or 3 carbon atoms bearing 1 to 3 substituents $R^6$ which are not defined as H. By contrast, it is to be noted that in the general method schemes below, the $R^6$ groups are used as if their definition includes H, to show where such $R^6$ groups may exist in the structures, and for ease in drawing. No change in the definition of $R^6$ is intended by this non-standard usage, however. Thus, only for purposes of the general method schemes below, $R^6$ may be H in addition to the moieties set forth in the definition of $R^6$. The ultimate compounds contain 1 to 3 non-hydrogen groups $R^6$.

**General Method A -** The key intermediates in which the rings A and B are substituted phenyl and phenylene respectively are conveniently prepared by use of a Friedel-Crafts reaction of a substituted biphenyl II with an activated acyl- containing intermediate such as the succinic or glutaric anhydride derivative III or acid chloride IV in the presence of a Lewis acid catalyst such as aluminum trichloride in an aprotic solvent such as 1,1,2,2-tetrachloroethane. The well known Friedel-Crafts reaction can be carried out with many alternative solvents and acid catalysts as described by E. Berliner, *Org. React.,* 5, 229 (1949) and H. Heaney, *Comp. Org. Synth.,* 2, 733 (1991).

Method A

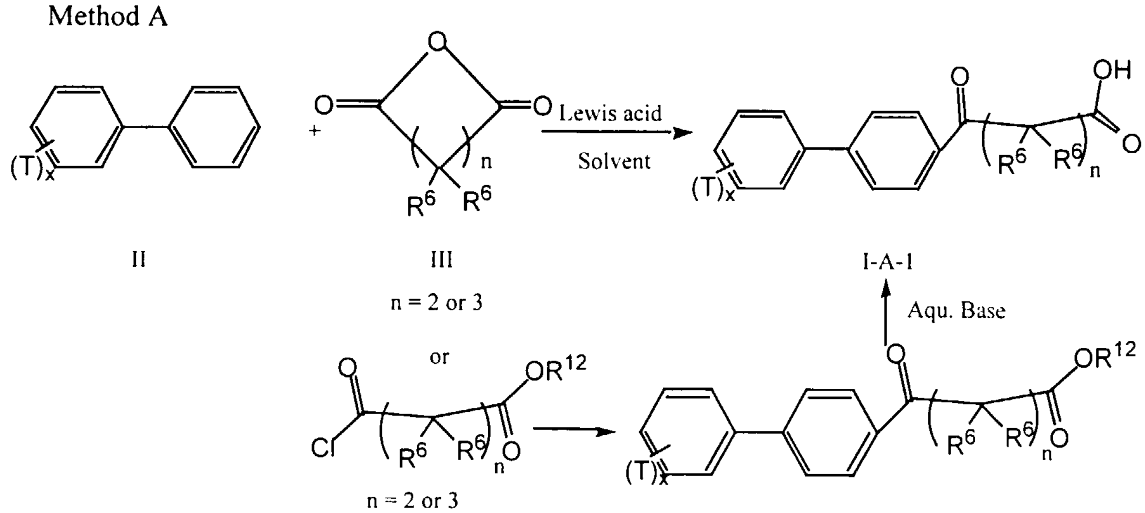


II

III

n = 2 or 3

or

I-A-1

n = 2 or 3

IV

I-A-2

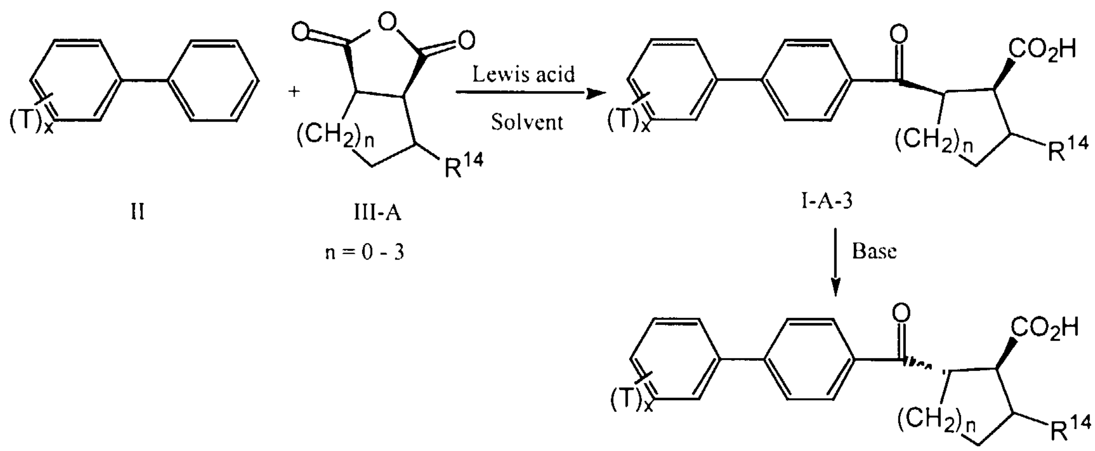


II

III-A

n = 0 - 3

I-A-3

I-A-4

If the anhydride III is monosubstituted or multiplesubstituted in an unsymmetrical way, the raw product I-A often exists as a mixture of isomers via attack of the anhydride from either of the two carbonyls. The resultant isomers can be separated into pure forms by crystallization or chromatography using standard methods known to those skilled in the art.

When they are not commercially available, the succinic anhydrides III can be prepared via a Stobbe Condensation of a dialkyl succinate with an aldehyde or ketone (resulting in side chain $R^6$), followed by catalytic hydrogenation, hydrolysis of a hemiester intermediate to a diacid and then conversion to the anhydride III by reaction with acetyl chloride or acetic anhydride. Alternatively, the hemiester intermediate is converted by treatment with thionyl chloride or oxalyl chloride to the acid chloride IV in which $R^{12}$ is lower alkyl. For a review of the Stobbe condensation, including lists of suitable solvents and bases see W.S. Johnson and G.H. Daub, *Org. React.*, **6**, 1 (1951). This method, as applied to the preparation of III ($R^6$ = H, isobutyl and H, n-pentyl), has been described by D. Wolanin, et al., US Patent 4,771,038, Sep. 13, 1988.

Method A is especially useful for the preparation of cyclic key intermediates such as I-A-3 in which two $R^6$ groups are connected in a methylene chain to form a 4-7 membered ring. Small ring (3-5 member) anhydrides are readily available only as cis isomers which yield cis invention compounds I-A-3. The trans compounds I-A-4 are then prepared by treatment of I-A-3 with a base such as DBU in THF.

The substituted four member ring starting material anhydrides such as III-A-1 are formed in a photochemical 2+2 reaction as shown below. This method is especially useful for the preparation of compounds in which $R^{14}$ is acetoxy or acetoxymethylene. After the subsequent Friedel-Crafts reaction the acetate can be removed by basic hydrolysis and the carboxyl protected by conversion to 2-(trimethylsilyl)ethyl ester. The resultant intermediate with $R^{14}$ = $CH_2OH$ can be converted to key intermediates with other $R^{14}$ groups by using procedures described in General Method K.

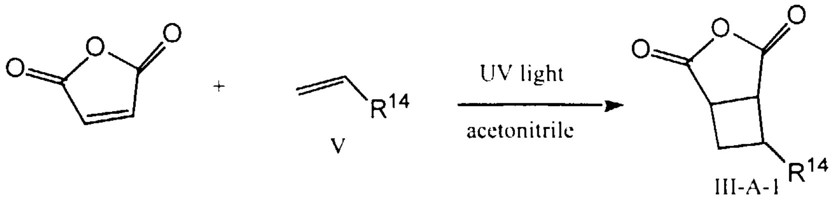


V

III-A-1

The Friedel Crafts method is also useful when double bonds are found either between C-2 and C-3 of a succinoyl chain (from maleic anhydride or 1-cyclopentene-1,2-dicarboxylic anhydride, for example) or when a double bond is found in a side chain, such as in the use of itaconic anhydride as starting material to yield products in which two $R^6$ groups as found on one chain carbon together form an exomethylene (=$CH_2$) group. Subsequent uses of these compounds are described in Methods D and E.

**General Method B -** Alternatively key intermediates can be prepared via a reaction sequence involving mono-alkylation of a dialkyl malonate VI with an alkyl halide to form intermediate VII, followed by alkylation with a halomethyl biphenyl ketone VIII to yield intermediate IX. Compounds of structure IX are then hydrolyzed with aqueous base and then heated to decarboxylate the malonic acid intermediate and yield I-B-2 (Method B-1). By using one equivalent of aqueous base the esters I-B-2 with $R^{12}$ as alkyl are obtained, and using more than two equivalents of base the acid compounds ($R^{12}$ = H) are obtained. Optionally, heat is not used and the diacid or acid-ester I-B-1 is obtained. Alternatively, the diester intermediate IX can be heated with a strong acid such as concentrated hydrochloric acid in acetic acid in a sealed tube at about 110 °C for about 24 hr to yield I-B-2 ($R^{12}$ = H).

Alternatively, the reaction of VI with VIII can be conducted before that with the alkyl halide to yield the same IX (Method B-2).

Intermediates VIII are formed from biphenyls II in a Friedel-Craft reaction with haloacetyl halides such as bromoacetyl bromide or chloroacetyl chloride. Alternatively, the biphenyl can be reacted with acetyl chloride or acetic anhydride and the resultant product halogenated with, for example, bromine to yield intermediates VIII (X = Br).

Method B has the advantage of yielding single regio isomers whereas Method A yields mixtures. Method B is especially useful when the side chains $R^6$ contain aromatic or heteroaromatic rings that may participate in intramolecular acylation reactions to give side products if Method A were to be used. This method is also very useful when the $R^6$ group adjacent to the carboxyl of the final compound contains heteroatoms such as oxygen, sulfur, or nitrogen, or more complex functions such as imide rings.

**Method B**

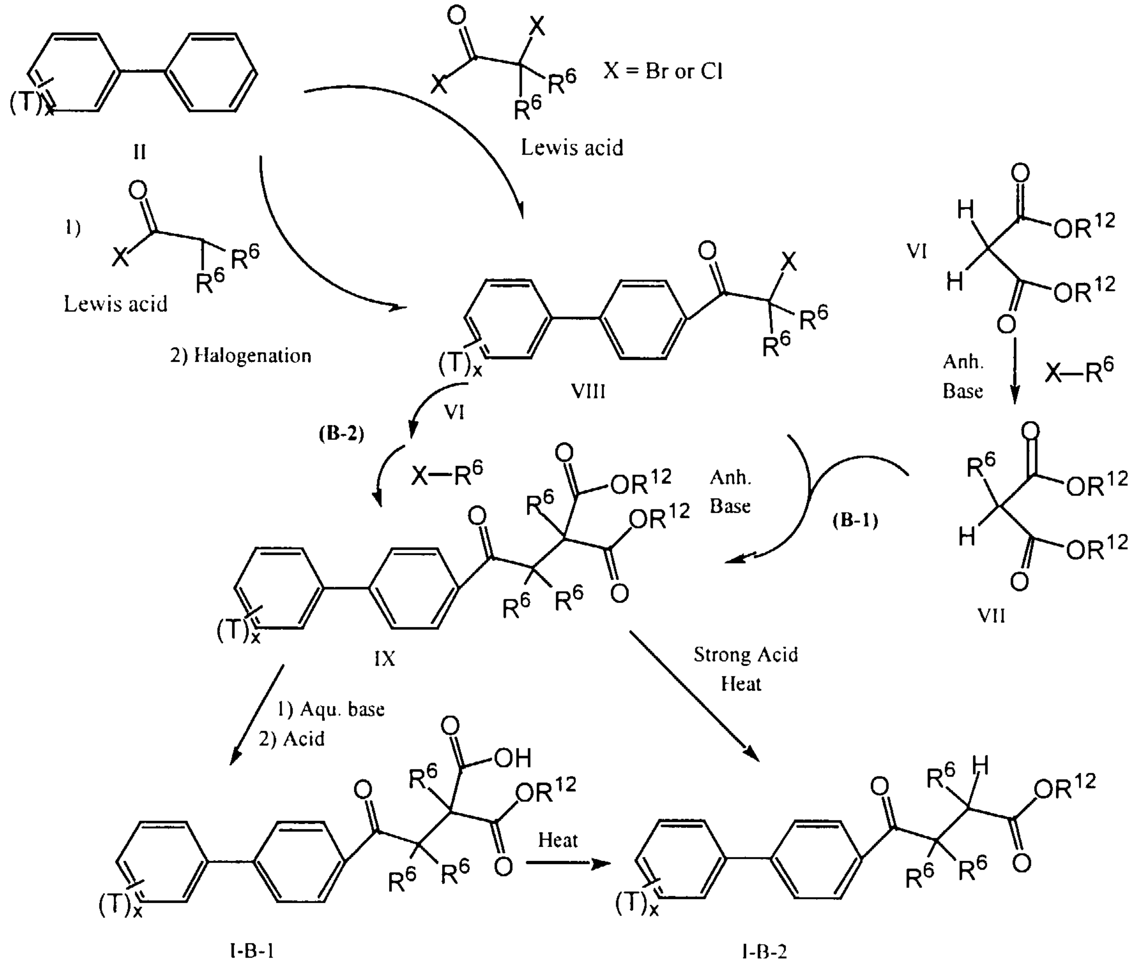


**General Method C -** Especially useful is the use of chiral HPLC to separate the enantiomers of racemic key intermediate mixtures (see, for example, D. Arlt, B. Boemer, R Grosser and W. Lange, *Angew. Chem. Int. Ed. Engl. 30 (1991) No. 12*). The key intermediates are prepared as pure enantiomers by use of a chiral auxiliary route - see, for example: D.A. Evans, Aldrichimica Acta, 15(2), 23 (1982) and other similar references known to one skilled in the art.

**C-1.** Acid halide X is reacted with the lithium salt of chiral auxiliary XI (R is often isopropyl or benzyl) to yield intermediate XII, which in turn is alkylated at low temperatures (typically under -50°C) with halo-tert-butylacetyl compound XIII to yield pure isomer XIV. The use of opposite chirality XI yields opposite chirality XIV. Conversion of XIV to the enantiomerically pure diacid XV is accomplished by treatment with lithium hydroxide/hydrogen peroxide in THF/water, followed by acids such as trifluoroacetic acid. The compound XV is then converted to enantiomerically pure anhydride III-A by treatment with acetyl chloride. The use of a Friedel-Crafts reaction as in method A then converts III-A to I-C-1.

**C-2.** Biphenyl starting material II may also first be reacted in a Friedel-Crafts reaction as earlier described with succinic anhydride followed by Fisher esterification with a lower alcohol such as methanol in the presence of a strong acid such as sulfuric acid to form acyl derivative I-C-2. The carbonyl group of this material is then blocked as a ketal such as that formed by treatment with 1,2-bistrimethylsilyloxyethane in the presence of a catalyst such as trimethylsilyltriflate in a suitable solvent. Many other ketal derivatives and reaction conditions familiar to those skilled in the art can also be used in this step. Basic hydrolysis of the ester followed by reaction of the resultant I-C-3 with XI in the presence of an amide coupling agent such as 1-(3-dimethylaminopropyl)-3-ethylcarbodiimide yields amide I-C-4. Reaction of this chiral amide with an alkylating agent such as alkyl or arylalkyl triflate or halide yields enantiomerically enriched product I-C-5 which can be converted to ketal acid I-C-6 by treatment with a weak base such as lithium hydroxide/hydrogen peroxide and then to keto acid I-C-7 by treatment with an acid. These deblocking steps can be conducted in either order.

## Method C-1

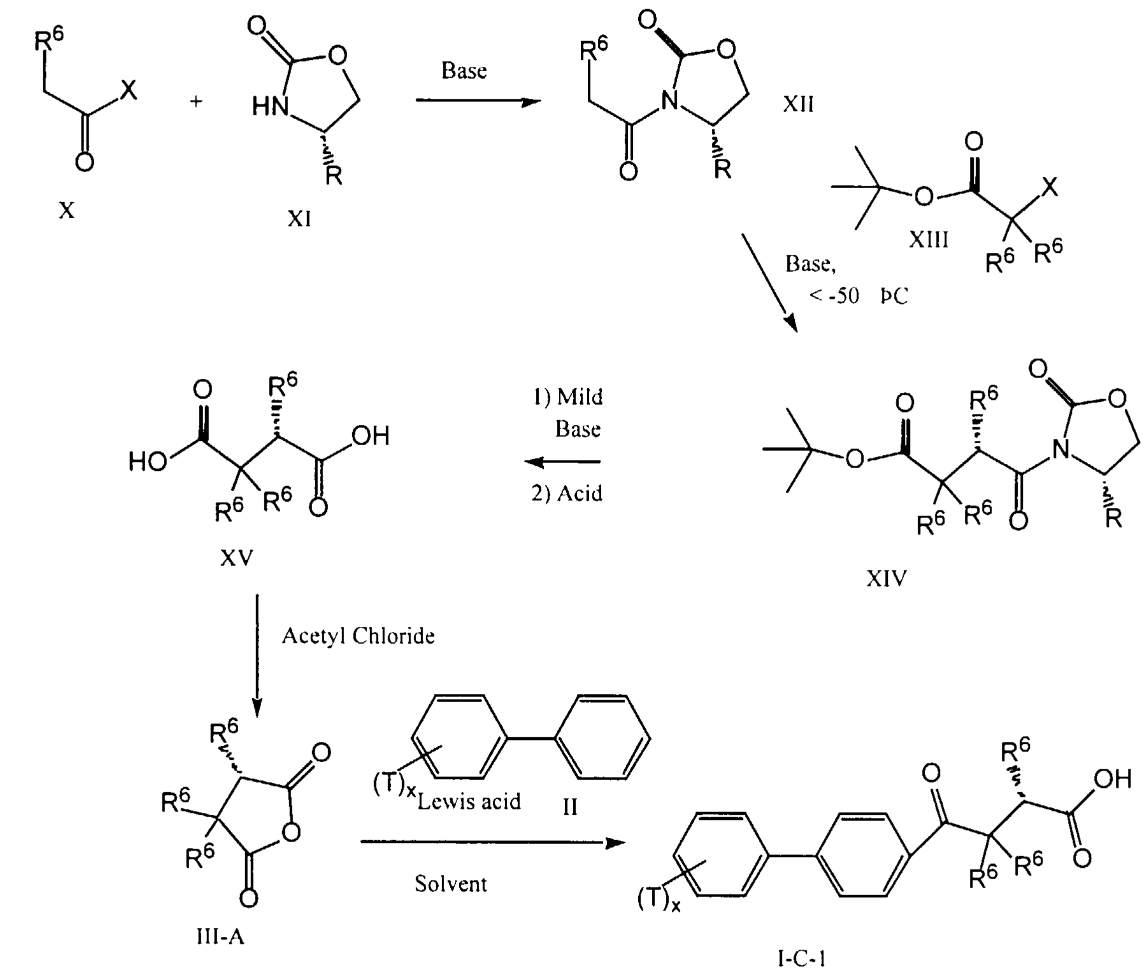
X
XI
Base
XII
XIII
Base,
< -50 ÞC
XIV
1) Mild
Base
2) Acid
XV
Acetyl Chloride
III-A
Lewis acid
II
Solvent
I-C-1

Method C-2

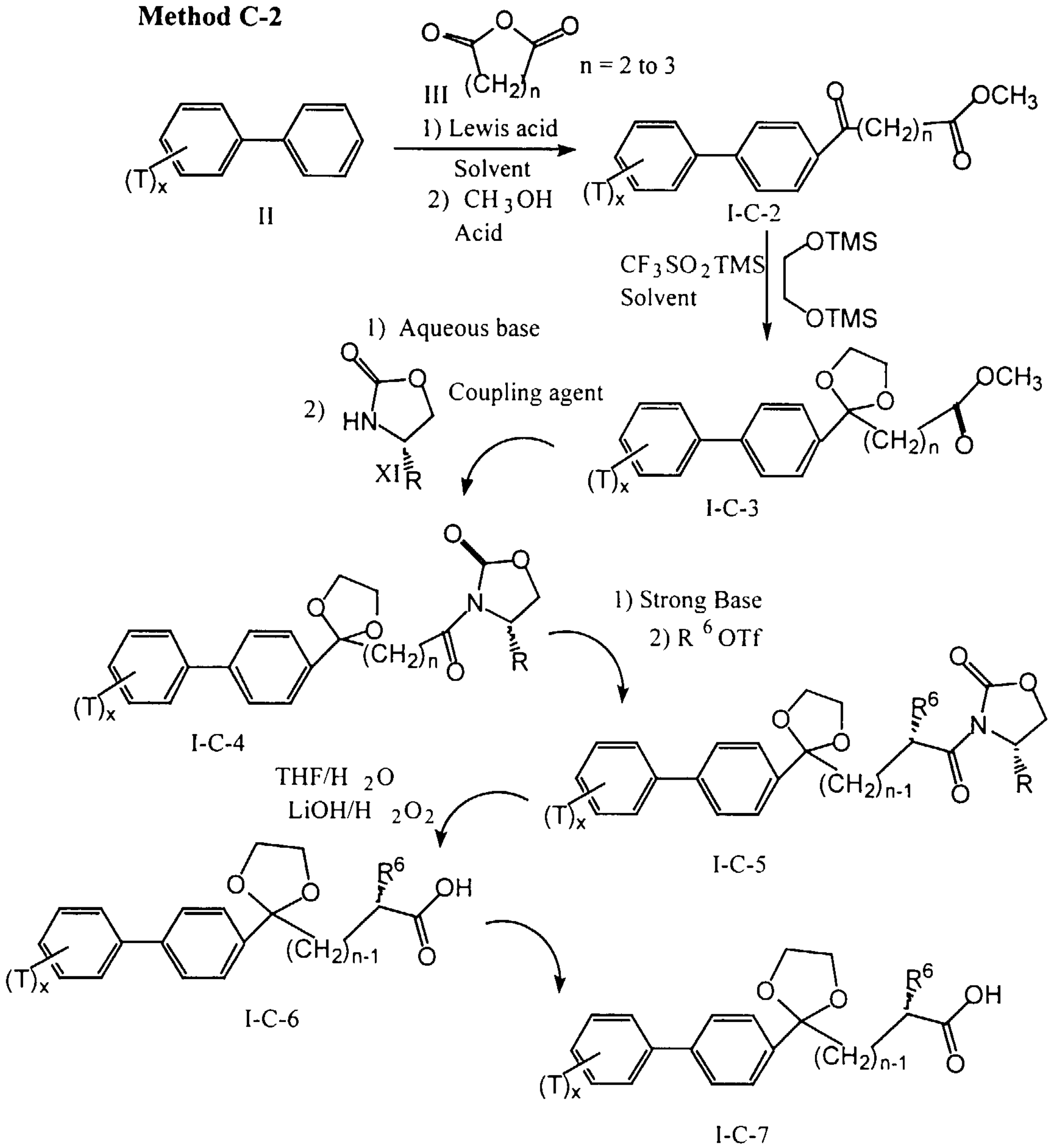


**General Method D -** Key intermediates in which $R^6$ are alkyl- or aryl- or heteroaryl- or acyl- or heteroarylcarbonyl-thiomethylene are prepared by methods analogous to those described in the patent publication WO 90/05719. Thus substituted itaconic anhydride XVI (n = 1) is reacted under Friedel-Crafts conditions to yield acid I-D-1 which can be separated by chromatography or crystallization from small amounts of isomeric I-D-5. Alternatively, I-D-5 are obtained by reaction of key intermediates I-D-4 (from any of Methods A through C) with formaldehyde in the presence of a base.

Compounds I-D-1 or I-D-5 are then reacted with a mercapto derivative XVII or XVIII in the presence of a catalyst such as potassium carbonate, ethyldiisobutylamine, tetrabutylammonium fluoride or free radical initiators such as azobisisobutyronitrile (AIBN) in a solvent such as dimethylformamide or tetrahydrofurane to yield key intermediates I-D-2, I-D-3, I-D-6 or I-D-7.

## Method D

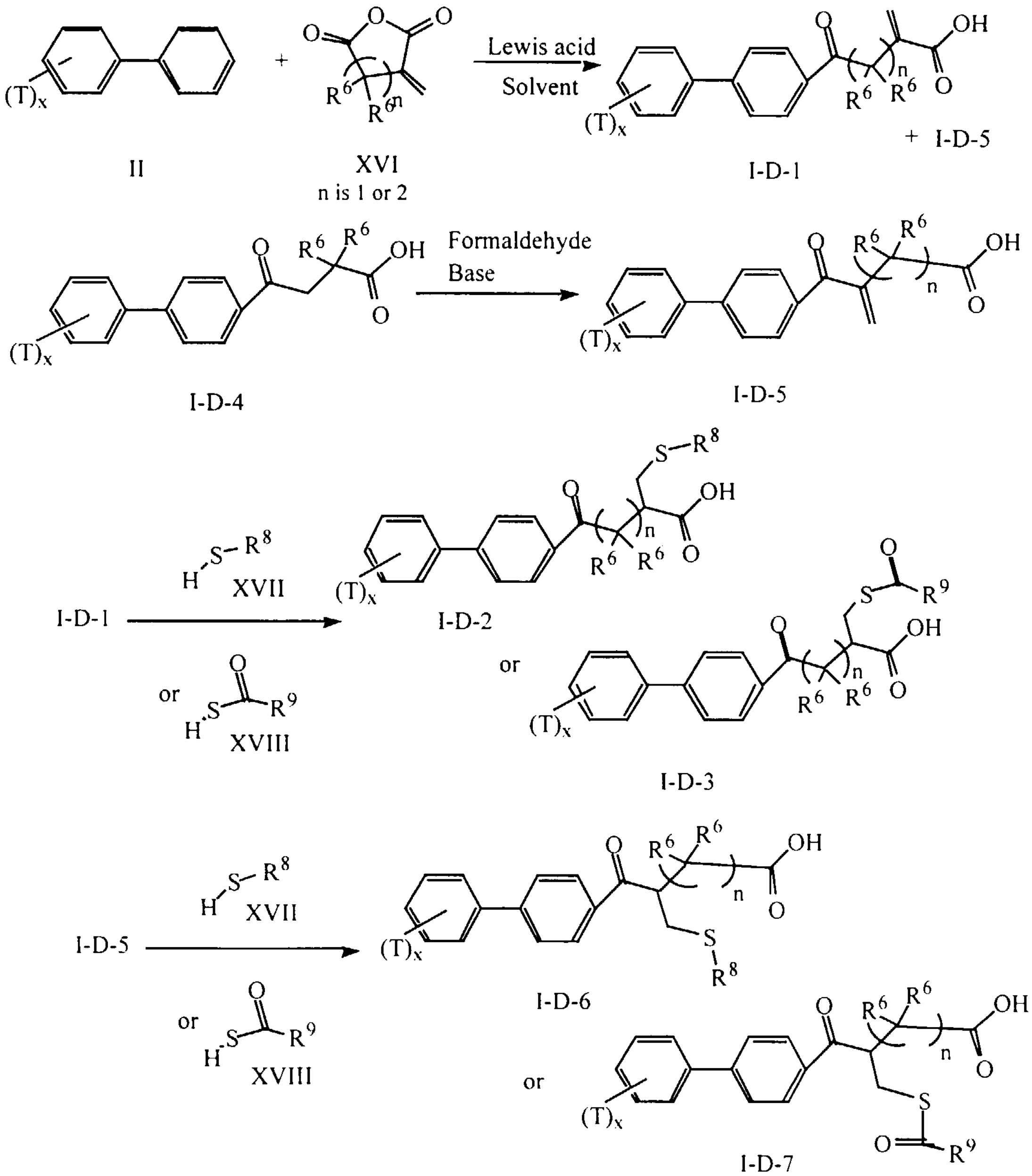


II, XVI (n is 1 or 2), I-D-1 + I-D-5, I-D-4, I-D-5, XVII, XVIII, I-D-2, I-D-3, I-D-6, I-D-7

**General Method E -** Reaction of optionally substituted maleic anhydride XIX under Friedel-Crafts conditions with II yields key intermediate I-E-1, which in turn is reacted with either of mercapto derivatives XVII or XVIII to yield key intermediates I-E-2 or I-E-3, or with substituted amine XX to yield key intermediate I-E-4. Esterification of I-E-1 (R6 = H) with $CH_3I$/DBU followed by reagent XXI and AgF and then basic hydrolysis yields pyrrolidine key intermediate I-E-5. $R^{14}$ can be various alkyl or arylalkyl groups including benzyl. Reaction of the intermediate ester (from step 2) with benzyloxycarbonyl chloride in THF at reflux followed by hydrolysis yields key intermediates in which $R^{14}$ is benzyloxycarbonyl.

**Method E**

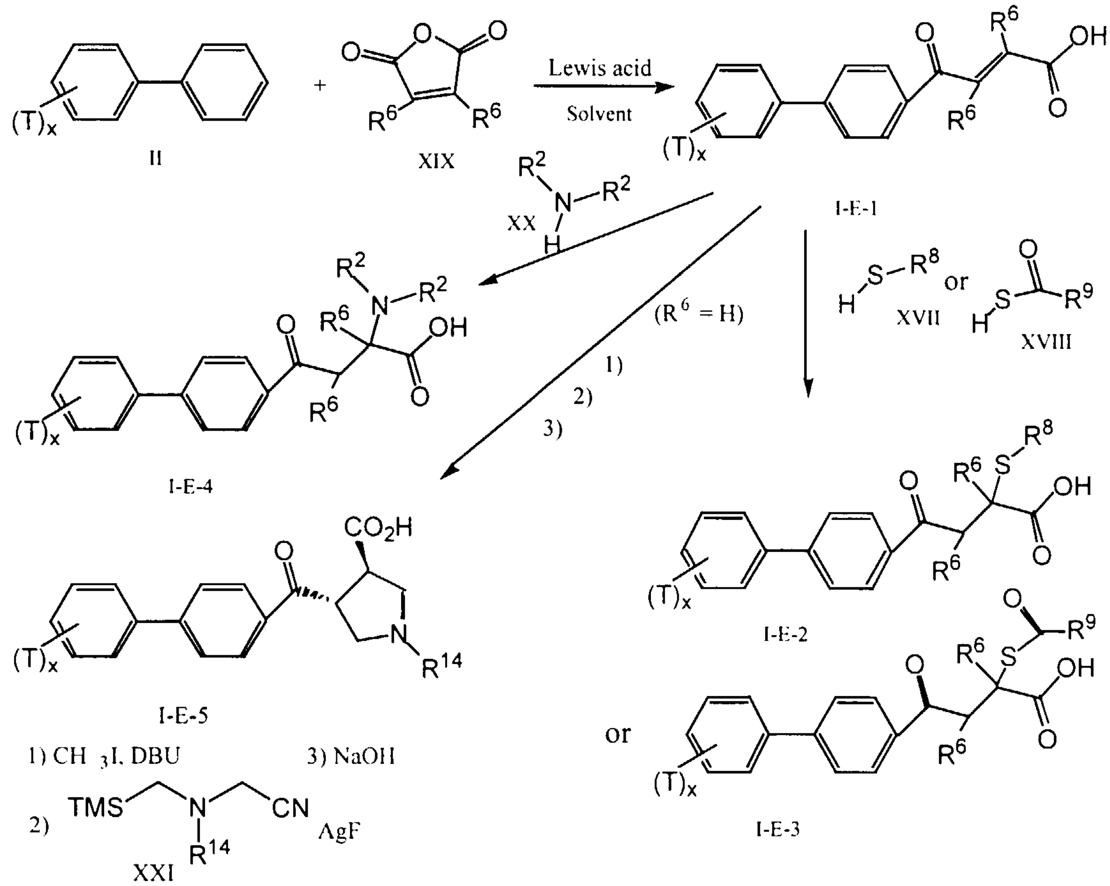


**General Method F -** Biaryl key intermediates such as those of this application may also be prepared by Suzuki or Stille cross-coupling reactions of aryl or heteroaryl metallic compounds in which the metal is zinc, tin, magnesium, lithium, boron, silicon, copper, cadmium or the like with an aryl or heteroaryl halide or triflate (trifluoromethane-sulfonate) or the like. In the equation below either Met or X is the metal and the other is the halide or triflate. Pd(com) is a soluble complex of palladium such as tetrakis(triphenylphosphine)-palladium(0) or bis-(triphenylphos-phine)-palladium(II) chloride. These methods are well known to those skilled in the art. See, for example, A. Suzuki, Pure Appl. Chem., 66, 213 - 222 (1994); A. Suzuki, Pure Appl. Chem., 63, 419 - 422 (1991); and V. Farina and G. Roth, "Metal-Organic Chemistry" Volume 5 (Chapter 1), 1994.

The starting materials XXIII (B = 1,4-phenylene) are readily formed using methods analogous to those of methods A, B or C but using a halobenzene rather than a biphenyl as starting material. When desired, the materials in which X is halo can be converted to those in which X is metal by reactions well known to those skilled in the art such as treatment of a bromo intermediate with hexamethylditin and palladium tetrakistriphenylphosphine in toluene at reflux to yield the trimethyltin intermediate. The starting materials XXIII (B = heteroaryl) are most conveniently prepared by method C but using readily available heteroaryl rather than biphenyl starting materials. The intermediates XXII are either commercial or easily prepared from commercial materials by methods well known to those skilled in the art.

These general methods are useful for the preparation of key intermediates for which Friedel-Crafts reactions such as those of Methods A, B, C, D or E would lead to mixtures with various biaryl acylation patterns. Method F is also especially useful for the preparation of key intermediates in which the aryl groups A or B contain one or more heteroatoms (heteroaryls) such as those compounds that contain thiophene, furan, pyridine, pyrrole, oxazole, thiazole, pyrimidine or pyrazine rings or the like instead of phenyls.

Method F

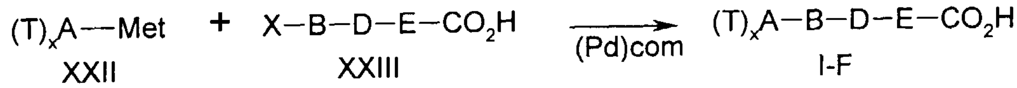


XXII XXIII I-F

T, x, A, B, E and D as in Structure I
Met = Metal and X = Halide or Triflate or
Met = Halide or Triflate and X = Metal

**General Method G -** When the $R^6$ groups of method F form together a 4 - 7 membered carbocyclic ring as in Intermediate XXV below, the double bond can be moved out of conjugation with the ketone group by treatment with two equivalents of a strong base such as lithium diisopropylamide or lithium hexamethylsilylamide or the like followed by acid quench to yield compounds with the structure XXVI. Reaction of XXVI with mercapto derivatives using methods analogous to those of General Method D then leads to key intermediate I-G-1 or I-G-2.

**Method G**

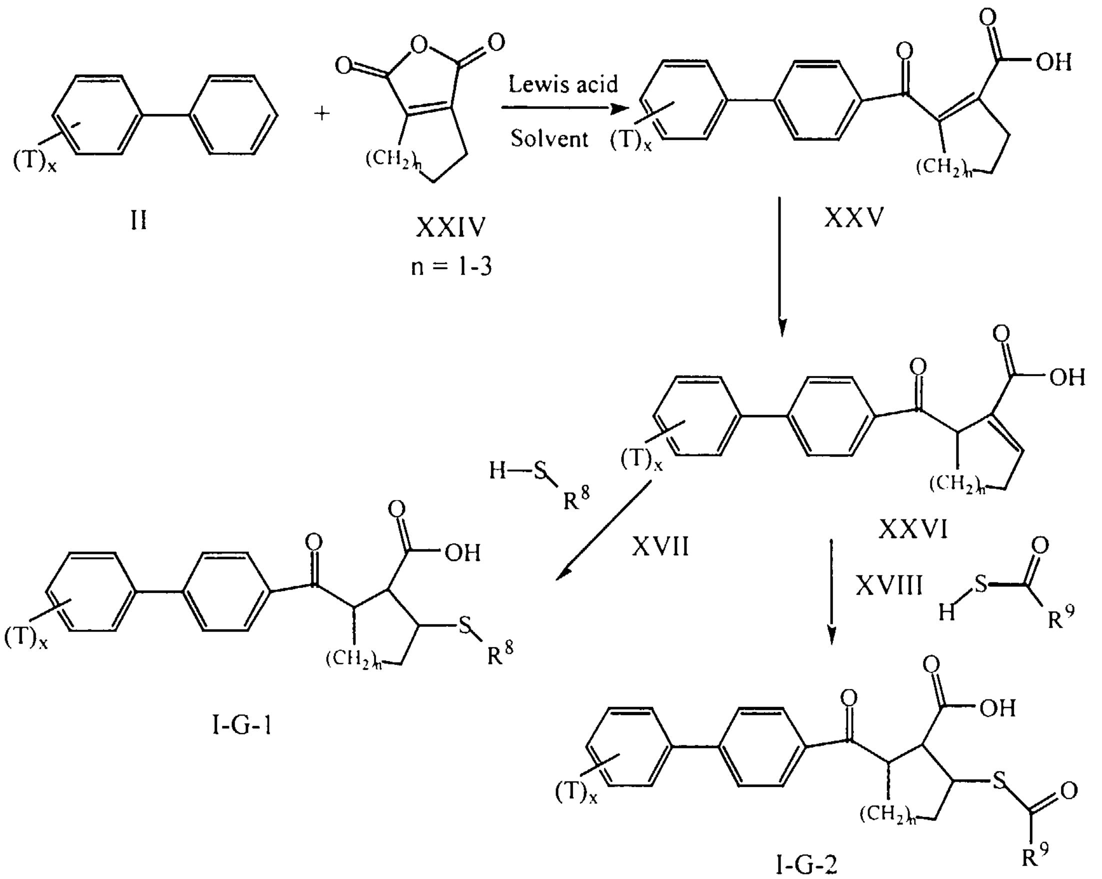


II, XXIV, XXV, XXVI, I-G-1, I-G-2

**General Method H -** Key intermediates in which two $R^6$ groups form a 4 - 7 member carbocyclic ring as in I-H below and $R^{14}$ is alkyl or arylalkyl are prepared according to method H. Starting material XXVII is reacted with two equivalents of a strong base such as lithium diisopropylamide (LDA) followed by an alkyl or arylalkyl halide ($R^{14}X$) to yield intermediate XXVIII. This material is then reduced to the alcohol with a reducing agent capable of selective reduction of the ketone such as sodium borohydride, followed by dehydration with triphenylphosphine / diethyl azodicarboxylate (DEAD) in a suitable solvent such as THF at reflux to yield XXIX. Hydrolysis of the ester with aqueous base followed by amide formation with $R^{12}ONHR^{12}$ (R is ($C_1$-$C_4$)-alkyl, but usually $CH_3$) in the presence of a coupling agent such as dicyclohexyldiimide (DCC) yields XXX. Other acyl activating groups well known to those skilled in the art such

as acid chlorides or mixed anhydrides could be used instead of XXX. Substituted biphenyl halide XXXI is reacted with an alkyl lithium such as two equivalents of t-butyl lithium to yield lithiated biphenyl XXXII which is then reacted with activated acyl compound XXX. The resultant intermediate XXXIII is then treated with diethylaluminum cyanide to yield intermediate XXXIV which is then hydrolyzed with aqueous acid to yield key intermediate I-H which is purified by chromatography on silica gel to afford pure isomers.

## Method H

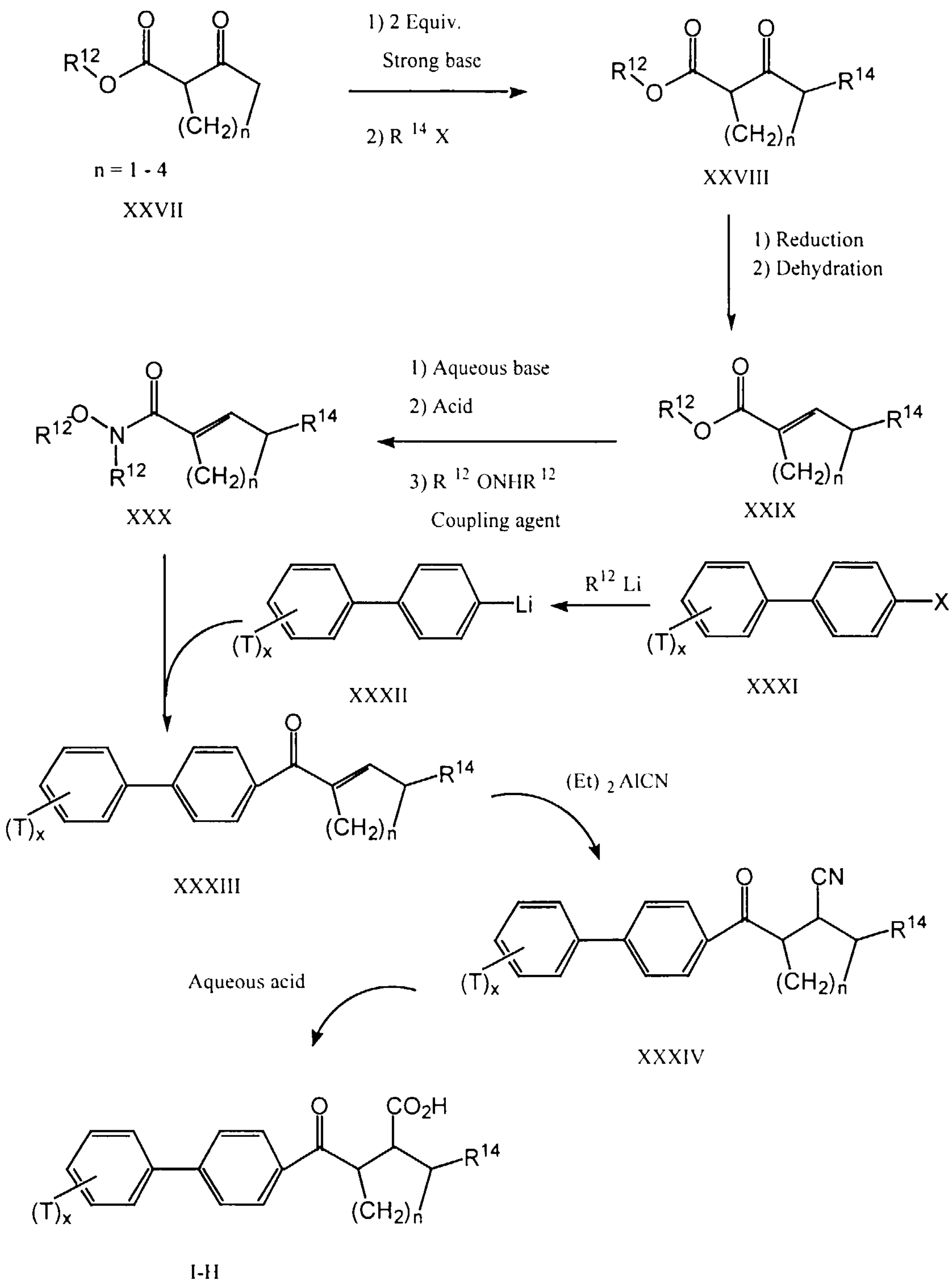


**General Method I -** Key intermediates in which two R6 groups together form a pyrrolidine ring are prepared

according to method I. Starting material XXXV (L-pyroglutaminol) is reacted under acid catalysis with benzaldehyde XXXVI (may be substituted) to yield bicyclic derivative XXXVII. A double bond is then introduced using phenylselenenyl methodology well known to those skilled in the art to yield XXXVIII, which, in turn, is reacted with a vinylcopper (I) complex to yield conjugate addition product XXXIX. Such reactions in which Lig can be, for example, another equivalent of vinyl group or halide are well known to those skilled in the art. Hydride reduction (lithium aluminum hydride or the like) of XXXIX followed by standard blocking with, for example, t-butyldimethylsilylchloride yields XXXX which in turn is reacted with an optionally substituted benzylchloroformate XXXXI to yield XXXXII. Ozonolysis of this intermediate followed by reductive workup (dimethylsulfide, zinc/acetic acid or the like) leads to aldehyde XXXXIII. Reaction of this aldehyde with a biphenyl organometallic such as XXXII yields alcohol XXXXIV. Deblocking of the silyl group with, for example, tetrabutylammonium fluoride followed by oxidation with, for example, pyridiniumdichromate or the like yields key intermediate 1-I-1 in which $R^{14}$ is a carbobenzyloxy group.

Alternatively the carbobenzyloxy group is removed by reaction with hydrogen and a catalyst such as palladium on carbon to yield the unsubstituted key intermediate 1-I-2 optionally followed by N-alkylation to yield key intermediate 1-I-3. These final steps are well known to those skilled in the art. Alternatively the intermediate XXXX can be directly treated with ozone followed by the other steps of this method to yield 1-I-3, in which $R^{14}$ is optionally substituted benzyl rather than as in 1-I-1.

This method is especially useful to prepare single enantiomers because starting material XXXV is available as either the isomer as drawn or as D-pyroglutaminol to yield enantiomeric products.

**Method I**

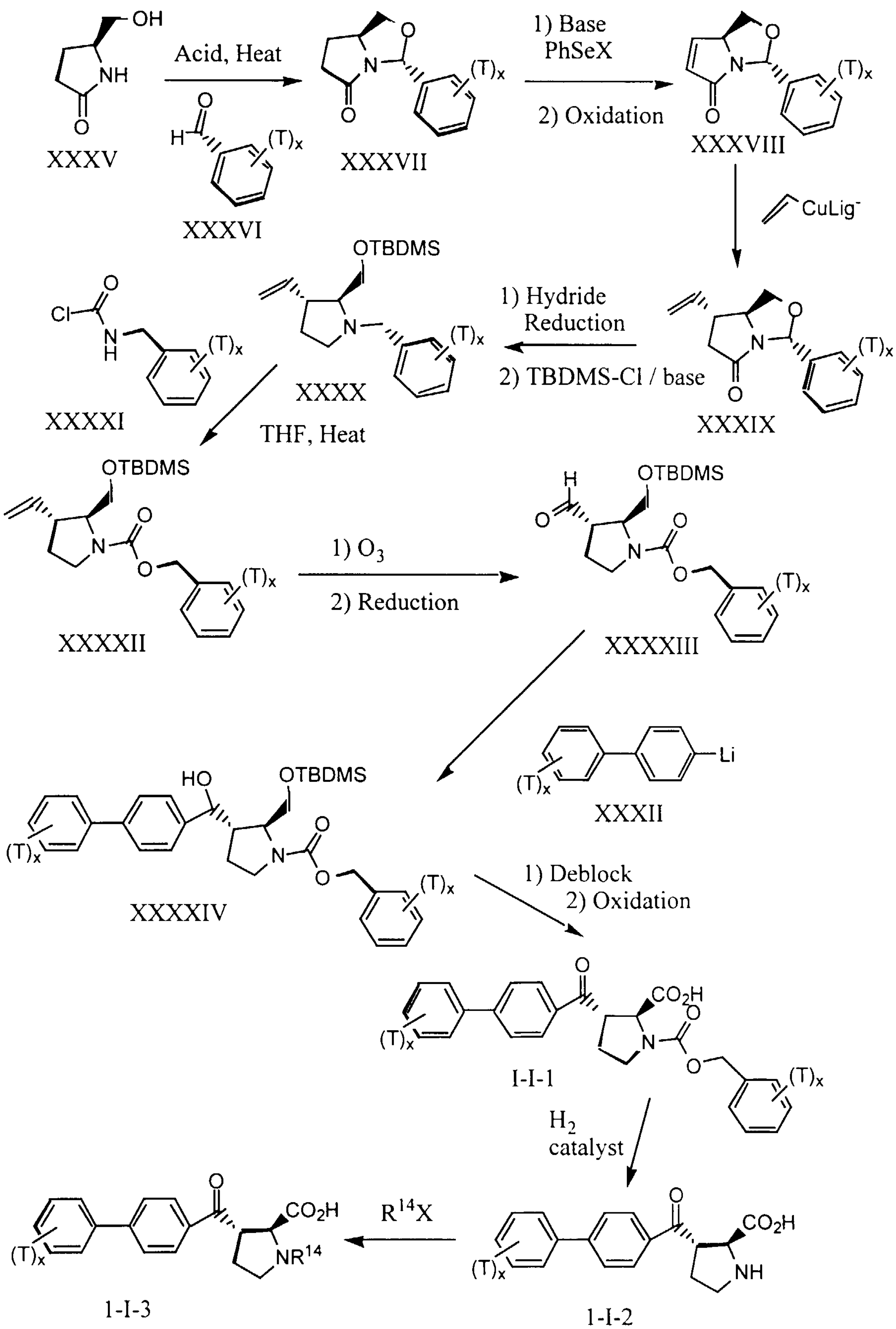
XXXV
Acid, Heat
XXXVI
XXXVII
1) Base
PhSeX
2) Oxidation
XXXVIII
CuLig⁻
XXXIX
1) Hydride
Reduction
2) TBDMS-Cl / base
XXXX
XXXXI
THF, Heat
XXXXII
1) $O_3$
2) Reduction
XXXXIII
XXXII
XXXXIV
1) Deblock
2) Oxidation
I-I-1
$H_2$
catalyst
1-I-2
$R^{14}X$
1-I-3

**General Method J -** The key intermediates in which E represents a substituted chain of 3 carbons are prepared by method J. Intermediates XXXXVII, if not available from commercial sources, are prepared by reaction of an activated biphenylcarboxylic acid derivative XXXXV with substituted acetic acid XXXXVI which has been converted to its bis-anion with two equivalents of a strong base such as LDA followed by heating to decarboxylate the intermediate keto acid. Product XXXXVII is then treated with methylenemalonate derivative XXXXVIII in the presence of a strong base such as sodium hydride to yield substituted malonate XXXXIX. This malonate can be further alkylated under conditions familiar to those skilled in the art to yield L which in turn is treated with acid and then heated to yield key intermediate I-J-1. Alternatively the final alkylation can be omitted to yield products in which the $R^6$ adjacent to the carboxyl is H. Alternatively XXXXVII can be alkylated with 3-halopropionate ester LI in the presence of base such as LDA to yield ester 1-J-2 which can then be hydrolyzed with aqueous base to yield key intermediate 1-J-3 upon treatment with acid. This method is especially useful if any of the groups $R^6$ contain aromatic residues.

## Method J

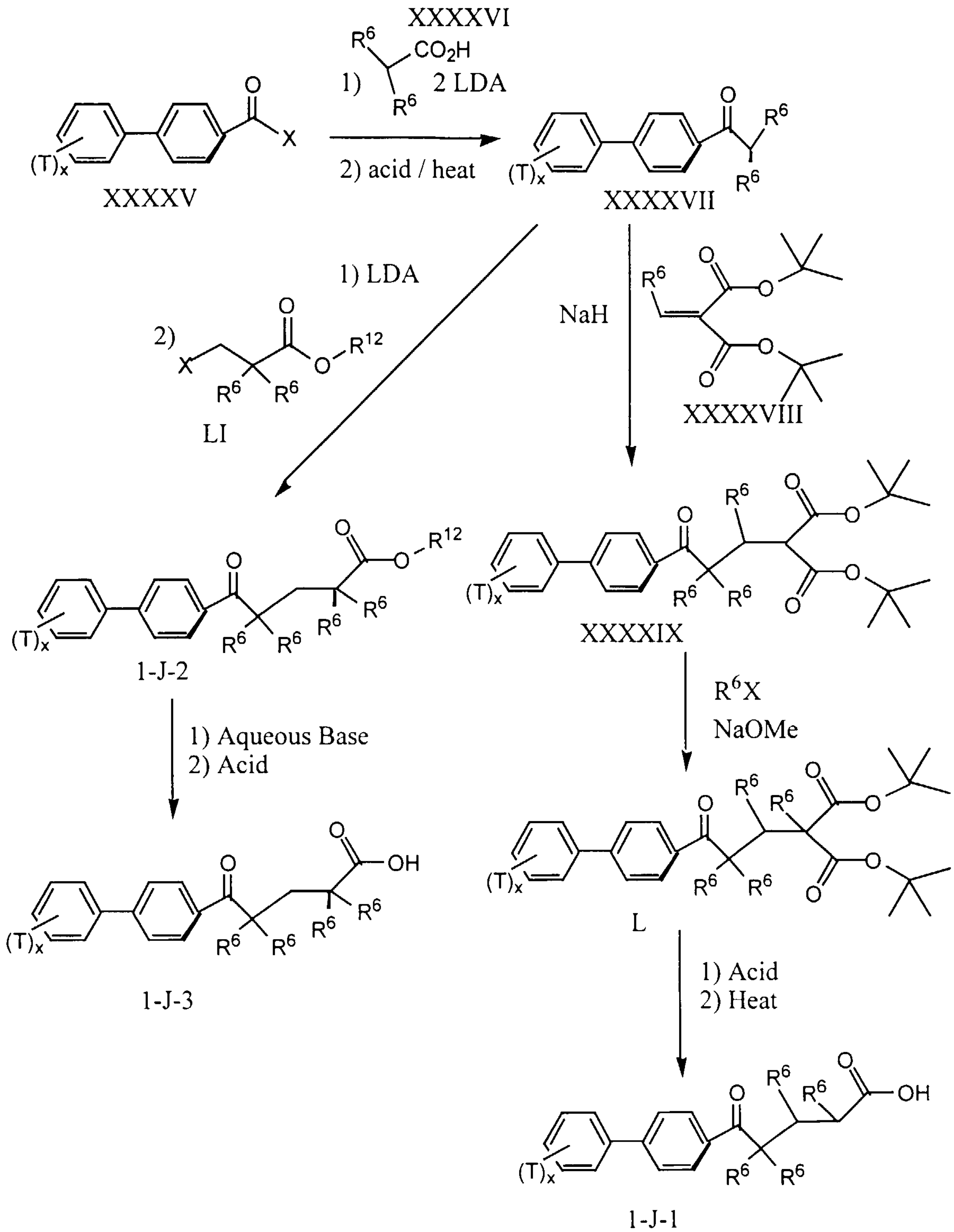


**Method K -** The key intermediates in which two $R^6$ groups are joined to form a substituted 5-member ring are most conveniently prepared by method K. In this method acid LII (R = H) is prepared using the protocols described in *Tetrahedron,* Vol. 37, Suppl., **1981**, 411. The acid is protected as an ester (R = benzyl or 2-(trimethylsilyl)ethyl) by use of coupling agents such as 1-(3-dimethylaminopropyl)-3-ethylcarbodiimide hydrochloride and procedures well known to those skilled in the art. Substituted bromobiphenyl LIII is converted to its Grignard reagent by treatment with magnesium which is then reacted with LII to yield alcohol LIV. Alcohol LIV is eliminated via base treatment of its mesylate

by using conditions well known to those skilled in the art to yield olefin LV. Alternatively LIII is converted to a trimethyltin intermediate via initial metallation of the bromide with n-butyllithium at low temperature (-78°C) followed by treatment with chlorotrimethyltin and LII is converted to an enoltriflate by reaction with 2-[N,N-bis(trifluoromethylsulfonyl)-amino]-5-chloropyridine in the presence of a strong aprotic base. The tin and enoltriflate intermediates are then coupled in the presence of a Pd° catalyst, CuI and $AsPh_3$ to yield directly intermediate LV. Ozonolysis of LV (workup with methyl sulfide) yields aldehyde LVI. Alternatively treatment with $OsO_4$ followed by $HIO_4$ converts LV to LVI.

Conversion of intermediate LVI to key intermediate I-K is accomplished in several ways depending on the identity of side chain function X. Reaction of LVI with Wittig reagents followed by hydrogenation yields products in which X is alkyl, aryl or arylalkyl. Reduction of aldehyde LVI with LAH yields alcohol I-K (X = OH). The alcohol is converted to phenyl ethers or N-phthalimidoyl compounds by use of the appropriate starting materials and Mitsunobu conditions well known to those skilled in the art; see O. Mitsunobu, Synthesis, 1 (1981). Alternatively the alcohol of I-K (X = OH) is converted to a leaving group such as tosylate (X = OTs) or bromide (X = Br) by conditions well known to those skilled in the art and then the leaving group is displaced by sulfur or azide nucleophiles to yield products with X = thioether or azide which in turn is reduced and acylated to yield amides (X = NHAcyl). Direct acylation of the alcohol I-K (X = OH) yields key intermediates in which X = OAcyl and reaction of the alcohol with various alkyl halides in the presence of base yields alkyl ethers (X = $OR^2$). In each case a final step is removal of acid blocking group R to yield acids (R = H) by using conditions which depend on the stability of R and X, but in all cases well known to those skilled in the art such as removal of benzyl by base hydrolysis or of 2-(trimethylsilyl)ethyl by treatment with tetrabutylammonium fluoride.

## Method K

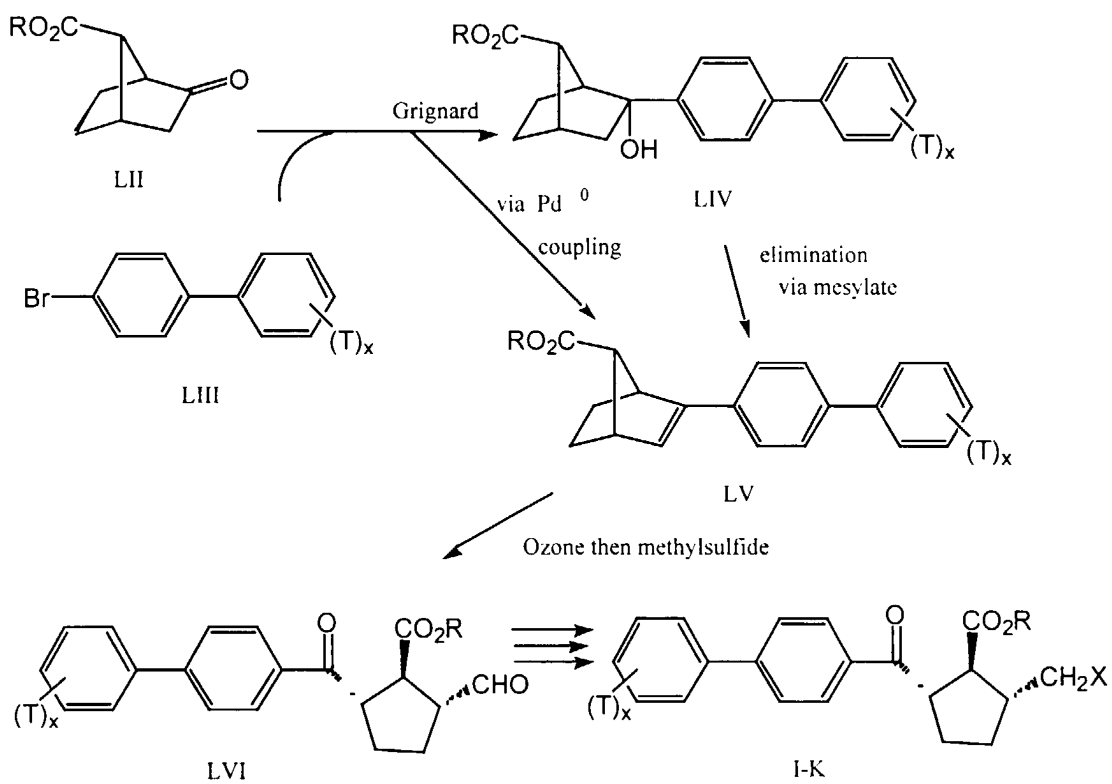


Suitable pharmaceutically acceptable salts of the compounds of the present invention that contain an acidic moiety include addition salts formed with organic or inorganic bases. The salt forming ion derived from such bases can be metal ions, e.g., aluminum, alkali metal ions, such as sodium of potassium, alkaline earth metal ions such as calcium or magnesium, or an amine salt ion, of which a number are known for this purpose. Examples include ammonium salts, arylalkylamines such as dibenzylamine and *N,N*-dibenzylethylenediamine, lower alkylamines such as methylamine, *t*-butylamine, procaine, lower alkylpiperidines such as *N*-ethylpipendine, cycloalkylamines such as cyclohexylamine or dicyclohexylamine, 1-adamantylamine, benzathine, or salts derived from amino acids like arginine, lysine

or the like. The physiologically acceptable salts such as the sodium or potassium salts and the amino acid salts can be used medicinally as described below and are preferred.

Suitable pharmaceutically acceptable salts of the compounds of the present invention that contain a basic moiety include addition salts formed with organic or inorganic acids. The salt forming ion derived from such acids can be halide ions or ions of natural or unnatural carboxylic or sulfonic acids, of which a number are known for this purpose. Examples include chlorides, acetates, tartrates, or salts derived from amino acids like glycine or the like. The physiologically acceptable salts such as the chloride salts and the amino acid salts can be used medicinally as described below and are preferred.

These and other salts which are not necessarily physiologically acceptable are useful in isolating or purifying a product acceptable for the purposes described below.

The salts are produced by reacting the acid form of the invention compound with an equivalent of the base supplying the desired basic ion or the basic form of the invention compound with an equivalent of the acid supplying the desired acid ion in a medium in which the salt precipitates or in aqueous medium and then lyophilizing. The free acid or basic form of the invention compounds can be obtained from the salt by conventional neutralization techniques, e.g., with potassium bisulfate, hydrochloric acid, sodium hydroxide, sodium bicarbonate, etc.

The compounds of the present invention are expected to inhibit the matrix metalloproteases MMP-1, MMP-2, MMP-3, MMP-8, MMP-9, MMP-12, MMP-13, and the related protease TACE, as well as the release of TNF$\alpha$ in vivo, and are therefore expected to be useful for treating or preventing the conditions referred to in the background section. As other MMPs not listed above share a high degree of homology with those listed above, especially in the catalytic site, it is deemed that compounds of the invention should also inhibit such other MMPs to varying degrees. Varying the substituents on the biaryl portions of the molecules, as well as those of the $R^6$ groups of the claimed compounds, is expected to affect the relative inhibition of the listed MMPs. Thus compounds of this general class can be "tuned" by selecting specific substituents such that inhibition of specific MMP(s) associated with specific pathological conditions can be enhanced while leaving non-involved MMPs less affected.

The method of treating matrix metalloprotease-mediated or TNF$\alpha$ release-mediated conditions may be practiced in mammals, including humans, which exhibit such conditions.

The inhibitors of the present invention are contemplated for use in veterinary and human applications. For such purposes, they will be employed in pharmaceutical compositions containing active ingredient(s) plus one or more pharmaceutically acceptable carriers, diluents, fillers, binders, and other excipients, depending on the administration mode and dosage form contemplated.

Administration of the inhibitors may be by any suitable mode known to those skilled in the art. Examples of suitable parenteral administration include intravenous, intraarticular, subcutaneous and intramuscular routes. Intravenous administration can be used to obtain acute regulation of peak plasma concentrations of the drug. Improved half-life and targeting of the drug to the joint cavities may be aided by entrapment of the drug in liposomes. It may be possible to improve the selectivity of liposomal targeting to the joint cavities by incorporation of ligands into the outside of the liposomes that bind to synovial-specific macromolecules. Alternatively intramuscular, intraarticular or subcutaneous depot injection with or without encapsulation of the drug into degradable microspheres e.g., comprising poly (DL-lactide-co-glycolide) may be used to obtain prolonged sustained drug release. For improved convenience of the dosage form it may be possible to use an i.p. implanted reservoir and septum such as the Percuseal system available from Pharmacia. Improved convenience and patient compliance may also be achieved by the use of either injector pens (e.g. the Novo Pin or Q-pen) or needle-free jet injectors (e.g. from Bioject, Mediject or Becton Dickinson). Prolonged zero-order or other precisely controlled release such as pulsatile release can also be achieved as needed using implantable pumps with delivery of the drug through a cannula into the synovial spaces. Examples include the subcutaneously implanted osmotic pumps available from ALZA, such as the ALZET osmotic pump.

Nasal delivery may be achieved by incorporation of the drug into bioadhesive particulate carriers (<200 $\mu$m) such as those comprising cellulose, polyacrylate or polycarbophil, in conjunction with suitable absorption enhancers such as phospholipids or acylcarnitines. Available systems include those developed by DanBiosys and Scios Nova.

Oral delivery may be achieved by incorporation of the drug into tablets, coated tablets, dragées, hard and soft gelatine capsules, solutions, emulsions or suspensions. Oral delivery may also be achieved by incorporation of the drug into enteric coated capsules designed to release the drug into the colon where digestive protease activity is low. Examples include the OROS-CT/Osmet™ and PULSINCAP™ systems from ALZA and Scherer Drug Delivery Systems respectively. Other systems use azo-crosslinked polymers that are degraded by colon specific bacterial azoreductases, or pH sensitive polyacrylate polymers that are activated by the rise in pH at the colon. The above systems may be used in conjunction with a wide range of available absorption enhancers.

Rectal delivery may be achieved by incorporation of the drug into suppositories.

The compounds of this invention can be manufactured into the above listed formulations by the addition of various therapeutically inert, inorganic or organic carriers well known to those skilled in the art. Examples of these include, but are not limited to, lactose, corn starch or derivatives thereof, talc, vegetable oils, waxes, fats, polyols such

as polyethylene glycol, water, saccharose, alcohols, glycerin and the like. Various preservatives, emulsifiers, dispersants, flavorants, wetting agents, antioxidants, sweeteners, colorants, stabilizers, salts, buffers and the like are also added, as required to assist in the stabilization of the formulation or to assist in increasing bioavailability of the active ingredient(s) or to yield a formulation of acceptable flavor or odor in the case of oral dosing.

The amount of the pharmaceutical composition to be employed will depend on the recipient and the condition being treated. The requisite amount may be determined without undue experimentation by protocols known to those skilled in the art. Alternatively, the requisite amount may be calculated, based on a determination of the amount of target enzyme which must be inhibited in order to treat the condition. It is expected that the compounds of the invention generally will be administered in doses in the range of 0.01-100 mg per kg of body weight per day.

The matrix metalloprotease inhibitors of the invention are useful not only for treatment of the physiological conditions discussed above, but are also useful in such activities as purification of metalloproteases and testing for matrix metalloprotease activity. Such activity testing can be both *in vitro* using natural or synthetic enzyme preparations or in vivo using, for example, animal models in which abnormal destructive enzyme levels are found spontaneously (use of genetically mutated or transgenic animals) or are induced by administration of exogenous agents or by surgery which disrupts joint stability.

**Biological Protocols**

Inhibitory activities of the compounds of the invention against matrix metalloproteases and production of TNF$\alpha$ may be determined as described below.

**Preparation of Gelatinase-B (MMP-9):**

MMP-9 is isolated modifying the previously described procedures of Hibbs et al (J. Biol. Chem., 260, 2493-2500, 1984) and Wilhelm et al (J. Biol. Chem., 264, 17213-17221, 1989). Briefly, polymorphonuclear leukocytes (PMN) preparations are isolated as described above from 3 or more units of freshly drawn whole blood. Cells are resuspended in phosphate buffered saline (PBS) containing 100 ng/ml phorbol myristate acetate (PMA) in the presence of 50 mM di-isopropylfluorophospate (DFP), 1 µg/ml leupeptin and aprotinin, and 1 mg/ml catalase for 1 hr at 37°C. Supernatants are collected by centrifugation (300 x g) and the samples are frozen at-70°C. All chromatographic methods are performed at 4°C. Thawed samples are concentrated 5-fold using an Amicon chamber equipped with a YM-10 membrane. The concentrate is pressure dialyzed against 0.02M Tris-HCl, 0.1 M NaCl, 1 mM $CaCl_2$ , 1 µM $ZnCl_2$, 0.001% Brij-35, 0.02% sodium azide ($NaN_3$), pH 7.5 and applied to DEAE ion exchange chromatography resin which is previously equilibrated with the same buffer at a flow rate of 0.4 ml/min. The column is extensively washed with the same buffer and gelatinase is eluted as 4 ml fractions from the column with 0.02M Tris-HCl, 0.5 M NaCl, 1 mM $CaCl_2$, 1 µM $ZnCl_2$, 0.001% Brij-35, 0.02% $NaN_3$, pH 7.5. Gelatinase containing fractions are observed by gelatin zymography (see below), loaded onto a gelatin agarose affinity resin and washed with the same buffer. Gelatinase activity is eluted at a flow rate of 1 ml/min from the column as 1 ml fractions with 0.02M Tris-HCl, 1 M NaCl, 1 mM $CaCl_2$, 1 µM $ZnCl_2$, 0.001% Brij-35, 0.02% $NaN_3$, pH 7.5 containing 10% dimethyl sulfoxide (DMSO). The fractions containing gelatinase activity are pooled and dialyzed against 0.005M Tris-HCl, 5mM NaCl, 0.5 mM $CaCl_2$ , 0.1 µM $ZnCl_2$, 0.001% Brij-35, pH 7.4. The protein content associated with material is determined with a micro-BCA assay (Pierce, Rockford, IL), lyophilized and reconstituted to a desired working concentration (100 µg/ml).

**Preparation of Gelatinase-A (MMP-2):**

Gelatinase A (MMP-2) is prepared using a vaccinia expression system according to the method of R. Fridman, et al., *J. Biol. Chem.,* 267, 15398 (1992).

**Preparation of Recombinant Truncated Prostromelysin (MMP-3):**

Truncated Prostromelysin-257 is expressed in a soluble form in E.coli as described by Marcy et al., Biochemistry, 30, 6476-6483, 1991. Soluble truncated prostromelysin is purified by a modification of the monoclonal antibody affinity chromatography method described by Housley et al., J. Biol. Chem., 268, 4481-87, 1993.

**P218 Quenched fluorescence Assay for MMP-3 Inhibition:**

This assay was originally described by Knight et al., FEBS Letters, 296, 263-266, 1992, for a related substrate. The assay is run continuously in a 3.0ml cuvette using a Perkin-Elmer LS 50 B Spectrofluorimeter at 25 °C in a final volume of 2.0 mls. P218 substrate (10mM) in 100% DMSO is diluted to a final concentration of 2.0 micromolar (µM)

into assay buffer: 50mM MES, pH 6.5 containing 150mM NaCl, 10mM CaCl2, 0.005% Brij-35, and 1%(v/v) DMSO. Test compounds(10mM) in DMSO are diluted in assay buffer at an initial concentration of 10 to 100 micromolar. These are diluted to a final concentration in the assay from 10 nM to 1 μM depending upon their potency previously determined in primary thiopeptilide assay described above. The reaction is initiated by the addition of recombinant stromelysin (MMP-3) at a final concentration of 1.0 nM. Upon peptide cleavage, the fluorescent MCA group is detected using an excitation wavelength of 328 nanometers and an emission wavelength of 393 nanometers. The assay is linear from 0.2 to 5nM MMP-3 concentration and percent inhibition is calculated as described above for the primary thiopeptilide assay and $IC_{50}$ values are determined by a linear regression analysis of percent inhibition versus log drug concentration. The peptide sequence of the MCA substrate, hereinafter designated P218, is shown below:

MCA-Pro-Lys-Pro-**Leu-Ala**-Leu-DPA-Ala-Arg-$NH_2$

P218

For MMP-3, this substrate has a $K_m$ of 16 μM at pH 6.5 and a kcat/$K_m$ value of 56,000$M^{-1}sec^{-1}$.

**P218 Quenched Fluorescence Assay for MMP-12 Inhibition:**

This assay is adapted from the one described by Knight et al., FEBS Letters, 296, 263-266 (1992) for MMP-3 and a related substrate. - The rate of hydrolysis of the synthetic substrate H-MCA-Pro-Lys-Pro-Leu-Ala-Leu-DPA-Ala-Arg-$NH_2$ (P218) by human recombinant MMP-12 is monitored fluorometrically, using an excitation wavelength of 340 nm and an emission wavelength of 395 nm, in the presence or absence of the test compounds. The assay is carried out in buffer containing 50 mM HEPES and 10 mM $CaCl_2$ at pH 7.0. The substrate is made up initially in 100% DMSO to a concentration of 1 x $10^{-2}$ M, then diluted in assay buffer to a final concentration of 20 μM. Test compounds (10 mM in DMSO) are diluted in assay buffer at an initial concentration of 0.3-1000 nM. These are diluted to a final concentration in the assay from 0.03 nM to 100 nM. The reaction is initiated by the addition of substrate at a final concentration of 20 μM. The total assay volume in a 96 well microtitre plate is 150 μl. Cleavage of the substrate between the Leu-Ala residues allows the fluorescence of the MCA group to be detected on a fluorometer (Cytofluor II) following excitation at 340 nm and emission at 395 nm. Change in fluorescence is continually monitored for a 40 min period.

The $K_i$'s are calculated using the method described by Williams and Morrison, Methods in Enzymology, 63, 437-467 (1979) to measure $K_{i\ apparent}$ for tight binding inhibitors, and is summarised as follows:

$$[I]_0/(1-v_i/v_0) = K_{i\ apparent} \times v_i/v_0 + [E]_0$$

$[I]_0$ and $[E]_0$ are inhibitor and enzyme concentrations, and $v_i$ / $v_0$ are reaction velocities with / without inhibitor. $[I]_0$ is equal to $IC_{50}$ when $v_i$ is half $v_0$, so that:

$$IC_{50} = 0.5 \times [E]_0 + K_{i\ apparent}$$

$IC_{50}$'s are determined at each enzyme concentration (0.044-0.98 μg/ml) using Xlfit software. $K_{i\ apparent}$ is then determined graphically from the plot of $IC_{50}$ versus MMP-12 concentration, using the intercepts to estimate $K_{i\ apparent}$. Thus, intercept values at $IC_{50}$ = 0 and $[E]_0$ = 0 are equal to -2 x $K_{i\ apparent}$ and $K_{i\ apparent}$, respectively. $IC_{50}$ values are calculated using % inhibition values at each enzyme concentration, ensuring data is taken from the linear part of the reaction rate curves. The $K_i$ can then be calculated from the equation:

$$K_i = K_{i\ apparent}/(1+S)/K_m$$

where S = substrate concentration (20 μM) and $K_m$ = dissociation constant (5.4 μM).

The following examples illustrate the selectivity of compounds of the invention or MMP-2 and MMP-9:

**Automated MMP Profiling Assay**

This assay is run with a protocol analogous to that reported for MMP-3 inhibition using the synthetic peptide P218 and each of the three enzymes and measuring quenched fluorescence. This assay can be run with each invention

compound with the three enzymes MMP-3, MMP-9 and MMP-2 in parallel as adapted for a 96-well microtitre plate using a Hamilton AT ® workstation.

The following examples illustrate the selectivity of compounds of the invention for MMP-2 and MMP-9:

| Example | MMP-1 $K_i$(nM) | MMP-2 $K_i$(nM) | MMP-3 $K_i$(nM) | MMP-9 $K_i$(nM) |
|---|---|---|---|---|
| 1 | 3,700 | 0.46 | 20 | 15 |
| 3 | 3,400 | 1.1 | 14 | 0.78 |
| 6 | 2,400 | 1.2 | 6.2 | 3.0 |

**LPS Induced TNFα Production in Mice**

The *in vivo* inhibitory properties of selected compounds can be determined using a murine LPS induced TNFα production *in vivo* model. BALB/c mice (Charles River Breeding Laboratories; Kingston, NY) in groups often are treated with either vehicle or compound. After one hour, endotoxin (E. coli lipopolysaccharide (LPS) 100 mg) is administered intraperitoneally (i.p.). After 90 min, animals are euthanized by carbon dioxide asphyxiation and plasma is obtained from individual animals by cardiac puncture into heparinized tubes. The samples are clarified by centrifugation at 12,500 x g for 5 min at 4 °C. The supernatants are decanted to new tubes, which are stored as needed at -20 °C. TNFα levels in sera are measured using a commercial murine TNF ELISA kit (Genzyme).

Other embodiments of the invention will be apparent to the skilled in the art from a consideration of this specification or practice of the invention disclosed herein. It is intended that the specification and examples be considered as exemplary only, with the true scope and spirit of the invention being indicated by the following claims.

**Middle cerebral artery occlusion model in rats**

**General:**

Wistar rats, weighting 200-250 g were used. Anaesthesia was induced with i.p. injection of 1.2 ml Ketavet (120 mg Ketamin) and 0.8 ml Rompun™ (2% Xylancin) per animal. Body temperatures were maintained at 37°C by means of a rectal thermometer and a heating system.

**Surgery:**

The MCA was occluded unilaterally according to the standard surgical procedure described by Bederson and colleagues with minor modifications. Briefly, the left temporal-parietal region of the head was shaved, and the skin was disinfected and opened between the orbit and the external ear canal. A midline incision was made, and the temporal muscle was divided and pulled aside with hooks to expose the lateral aspect of the skull. The facial nerve, major facial arteries and veins, the lateral eye muscle, the intra- and extra orbital lacrimal glands and the zygomatic bone were left intact. Under an operation microscope a small burr hole was drilled directly under the zygomatic arc, 1 to 2 millimeters rostral to its caudal origin.

After the dura was carefully opened, the exposed MCA and its branches were permanently occluded between the olfactory tract and the inferior cerebral vein by electro-coagulation (Bipolator 50, Fischer MET GmbH, Freiburg, Germany). To avoid recanalization, the occluded vessels were removed. The operation area was covered with a small piece of sterile absorbable gelatine sponge (Marbagelan, Behringwerke AG, Marburg, Germany). Muscle and skin wounds were closed with tissue glue (Histoacryl, B. Braun Melsungen AG, Melsungen, Germany). As it has been reported that hypothermia might act neuroprotective in animal models of ischemia [10], the body temperature was monitored during the surgery and maintained between 36.5 and 37.5°C by using a heating pad. The animals recovered from anesthesia, lying on a heating pad and covered with some layers of tissue. After recovery from anesthesia, the rats were returned to their home cage.

**Subdural hematoma (SDH) model in rats**

**General:**

Wistar rats, weighting 200-250 g were used. Anaesthesia was induced with i.p. injection of 1.2 ml Ketavet (120 mg Ketamin) and 0.8 ml Rompun™ (2% Xylancin) per animal. Body temperatures were maintained at 37°C by

means of a rectal thermometer and a heating system.

**Surgery:**

A midline scalp incision was made and the saggital and coronal sutures identified. A burr hole 3 mm in diameter was drilled 2 mm to the left of the saggital suture and 1 mm posterior to the coronal suture. Using an operating microscope, the dura was incised and a blunt, pre-manufactured plastic tip (ca. 6.0 mm long) was carefully inserted 0.8 mm deep into the subdural space. Rapid-curing cyanoacrylate glue (Histoacryl) was used to seal the burr hole and secure the position of the plastic tip. The subdural hematoma was created by slowly injecting 0.2 ml of non-heparinized, freshly drawn autologous venous blood into the subdural space over 4 minutes. The plastic tip was cut off flush with the scull and sealed with cyanoacrylate glue (Histoacryl). The scalp was sutured after application of sulphonamide powder and 2% lignocaine hydrochloride gel and the animals recovered from anesthesia, lying on a heating pad and covered with some layers of tissue. After recovery from anesthesia, the rats were returned to their home cage.

**Measurement and quantification of edema formation**

The edema formation was assessed 24 hours after surgery. The animals were killed by decapitation. The brain was removed and cut exactly at the midline. The wet weight of both hemispheres was determined and after drying for 24 hours at 110°C the dry weights were determined. Based on the increase in tissue water content, tissue swelling (i.e., increase in volume) may be calculated employing the following (modified) equation:

$$\frac{\Delta DW(\%)}{DW\varepsilon} \times 100(\% \mathit{Swelling})$$

where DW % is the difference in tissue dry weight between the normal and edematous brain, and DW the dry weight (%) of the edematous brain.

In the rat-SDH- model, Example 5 reduced the increase in tissue water content by 38% at a dose of 0.3 mg/kg i.v.

**Preparation Examples**

Examples 1-4 and 6-43 were essentially prepared as described in the indicated references.

**Example 5**

(+)-4-(4'-Chloro-biphenyl-4-yl)-4-oxo-2-[2-(4-oxo-4H-benzo[d][1,2,3]triazin-3-yl)-ethyl]-butyricacid

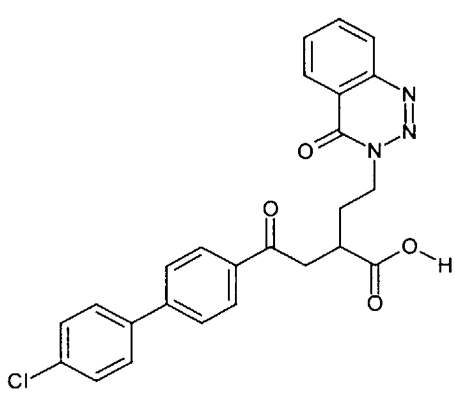

Example 6 was separated on a chiral HPLC-column prepared from monomer 2b described in Table 1 of Arlt et al. Angew.Chem.Int.Ed.Engl. 1991, 30, 1662-1664 to yield as the second eluting fraction the dextrorotatory enantiomer.

$[\alpha]_D^{20}$(c=0.872 g/100ml, THF) = + 15.30°

**Example 44**

4-(4'-Chloro-biphenyl-4-yl)-4-oxo-2-[2-(4-oxo-4H-benzo[d][1,2,3]triazin-3-yl)-ethyl]-butyricacid potassium

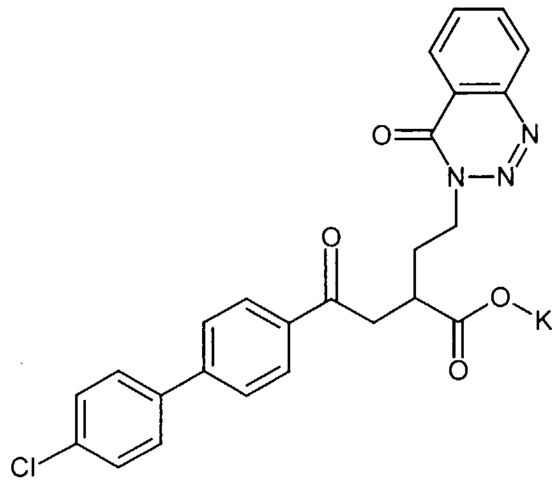

20mg of Example 6 were dissolved in 1ml THF and treated dropwise with a solution of 4.9mg KOtBu in 1ml THF. The resulting solution was stirred for 30min at room temperature. The solvent was removed in vacuo giving the potassium salt as an off white solid.

200 MHz $^1$H-NMR (DMSO-d6): 1.85, m, 1H; 2.10, m, 1H; 2.58, m, 1H; 2.72, m, 1H; 3.50, m, 1H; 4.47, m, 2H; 7.55, d, 2H; 7.74, d, 4H; 7.90, m, 1H; 7.98, d, 2H; 8.03, m, 1H; 8.18, m, 2H.

**Example 45**

4-(4'-Chloro-biphenyl-4-yl)-4-oxo-2-[2-(4-oxo-4H-benzo[d][1,2,3]triazin-3-yl)-ethyl]-butyricacid sodium

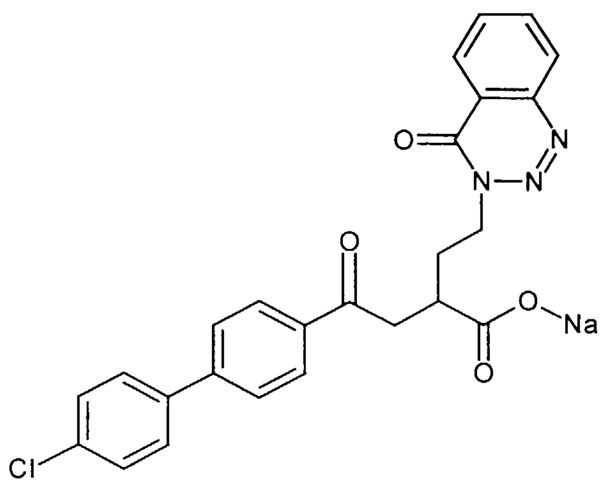

200mg of Example 6 were dissolved in 5ml THF and treated dropwise with a solution of 42mg NaOtBu in 2ml THF. The resulting solution was stirred for 30min at room temperature. The solvent was removed in vacuo giving the sodium salt as off white solid.

200 MHz $^1$H-NMR (DMSO-d6): 1.85, m, 1H; 2.13, m, 1H; 2.65, m, 2H; 3.52, m, 1H; 4.50, m, 2H; 7.55, d, 2H; 7.74, d, 4H; 7.90, m, 1H; 7.98, d, 2H; 8.03, m, 1H; 8.20, m, 2H.

## Example 46

(rac)-4-(4'-Ethoxy-biphenyl-4-yl)-2-[2-(4-oxo-4H-benzo[d][1,2,3]triazin-3-yl)-ethyl]-4-oxo-butyric acid

**Intermediate 46A**

4-Ethoxybiphenyl

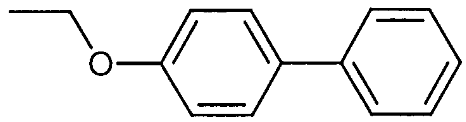

Iodoethane (68.7 g, 35,57 mL, 440.6 mmol) was added to a suspension of 50 g (170.2 mmol) 4-Hydroxybiphenyl and 40.6 g (293.75 mmol) $K_2CO_3$ in 600 mL acetone. The resulting reaction mixture was stirred under refluxe for 16 hours. After cooling to room temperature the acetone was removed under reduced pressure, the residue was dissolved in ethyl acetate and extracted with water. The aqueous layers where extracted 3 times with ethyl acetate, the combined organic phases dried ($Na_2SO_4$) and evaporated to yield 56 g of the desiered compound as a colorless solid.
Yield: 56 g (96 %)
$^1$H-NMR ($D_6$-DMSO): 7.55-7.65 (m, 4H), 7.42 (t, J=8 Hz, 2H), 7.3 (t, J=8Hz, 1H), 6.95-7.05 (m, 2H), 4.07 (q, J=7Hz, 2 H), 1.35 (t, J=7Hz, 3H)

**Intermediate 46B**

2-Bromo-4'-(4'-ethoxyphenyl)acetophenone

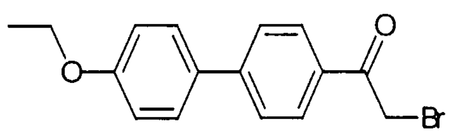

A solution of 18.54 g (93.51 mmol) of Intermediate 46A in 1.78 L $CH_2Cl_2$ was cooled to 0°Cand placed under argon. Bromoacetyl bromide (28.31 g, 12,22 mL, 140.27 mmol) in 2 mL $CH_2Cl_2$ was added, and then $AlCl_3$ (37.41 g, 280.53 mmol) was added in portions over 60 min. After the addition was complete, the mixture was stirred for 20 h, warming to rt. The mixture was then poured slowly into a stirred mixture of 1 N HCl/ $CH_2Cl_2$. The organic layer was separated, dried ($Na_2SO_4$) and evaporated. The crude product (31 g) was purified using flash chromatography (Hexane/ $CH_2Cl_2$ : 1/1).
Yield: 27.9 g (93 %)
$^1$H-NMR ($D_6$-DMSO): 8.08 (d, J=8Hz, 2H), 7.81 (d, J=8Hz, 2H), 7.73 (d, J=8Hz, 2Hz), 7.06 (d, J=8Hz, 2H), 4.95 (s, 2H), 4.1 (q, J=7Hz, 2H), 1.36 (t, J=7Hz, 3H)

**Intermediate 46C**

di(tert-butyl) 2-{2-[4-oxo-1,2,3-benzotriazin-3(4H)-yl]ethyl}malonate

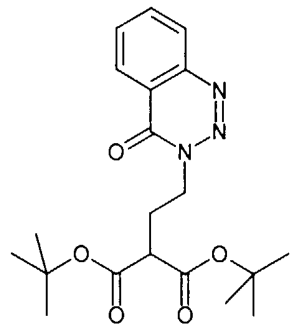

46.2g (177mmol) Di(tert-butyl) 2-(2-hydroxyethyl)malonat are dissolved in 600ml THF. 69.8g (266mmol) triphenylphosphin und 39.2g (266mmol) 1,2,3-benzotriazin-4(3H)-one are added. 46.4g (266mmol) DEAD are added dropwise. The reaction mixture was stirred overnight at roomtemperature. The solvent is removed in vacuo and the product obtained by chromatography (cyclohexan/ethylacetate 6:1).
Yield: 51.8g (63%).
200 MHz $^1$H-NMR ($CDCl_3$): 1.43, s, 18H; 2.43, quar., 2H; 3.30, t, 1H; 4.57, t, 2H; 7.80, m, 1H; 7.94, m, 1H; 8.16, dd, 1H; 8.36, dd, 1H.

**Intermediate 46D**

tert.-Butyl-4-(4'-ethoxy-biphenyl-4-yl)-4-oxo-2-(tert.-butyloxycarbonyl)-2-[2-(4-oxo-4H-benzo[d][1,2,3 ]triazin-3-yl)-ethyl]-butanoate

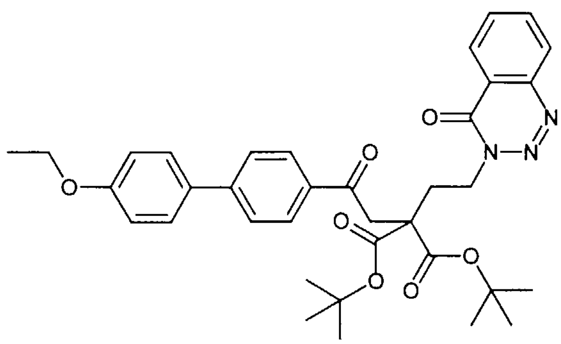

A solution of Intermediate 46C (1.29 g, 3.32 mmol) in 15 mL DMF was added dropwise to a suspension of NaH ( 170 mg, 4.15 mmol) in 5 mL DMF and stirred for 30 min at rt. Intermediate 46B (1.06 g, 3.32 mmol) in 15 ml DMF was added slowly and the resulting mixture was stirred for 2.5 h at rt. The reaction was quenched with saturated $NH_4Cl$ solution, extracted twice with diethyl ether, washed with saturated $NaHCO_3$, water and brine, dried ($Na_2SO_4$) and evaporated. The crude product was purified using flash chromatography (Hexane / Ethyl acetate : 9/1->5/1).
Yield: 1.34 g (59 %)
$^1$H-NMR ($D_6$-DMSO): 7.65-8.25 (m, 10H), 7.05 (d, J=8Hz, 2H), 4.39-4.51 (m, 2H), 4.1 (q, J=7Hz, 2H), 3,75 (s, 2H), 2.45-2.55 (m, 2H), 1.33-1.4 (m, 21H)

**Example 46**

(rac)-4-(4'-Ethoxy-biphenyl-4-yl)-2-[2-(4-oxo-4H-benzo[d][1,2,3]triazin-3-yl)-ethyl]-4-oxo-butyric acid

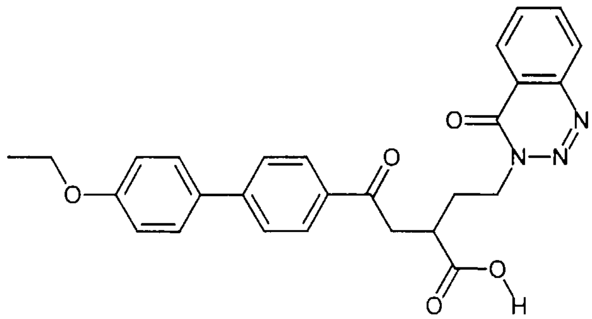

1.31 g (2.09 mmol) of Intermediate 46D were added in one portion to a cooled (0°C) 1:1 mixture of $CH_2Cl_2$ and trifluoroacetic acid (20 mL). The reaction mixture was stirred for 2 h at rt, evaporated and dried under vacuum. The residue was disolved in 20 mL dioxane and heated for 5 h under reflux. The rection mixture was evaporated, the residue triturated with ethylacetate, stirred for 15 min and filtered. The remaining solid was dried under vacuum.
Yield: 0.77 g (78 %)
$^1$H-NMR ($D_6$-DMSO): 12.35 (s, 1H), 8.18-8.3 (m, 2H), 7.9-8.15 (m,4H), 7.79 (d, J=8Hz, 2H), 7.61 (d, J=8Hz, 2H), 7.05 (d, J=8Hz, 2H), 4.42-4.65 (m, 2H), 4.1 (q. J=7Hz, 2H), 3.24-3.6 (m, 4H), 2.88-3.05 (m,lH), 2.02-2.35 (m, 2H), 1.38 (t, J=7Hz, 3H)

Example 46 was separated into pure enantiomers by HPLC on a chiral column.

## Example 47

First eluting enantiomer A
Yield: 168 mg (24 %)
$[\alpha]_D^{20}$(c= 0.66g/100ml, THF) = +17.05°

## Example 48

Second eluting enantiomer B:
Yield: 153 mg (22%)
$[\alpha]_D^{20}$(c= 0.58g/100ml, THF) = -18.78°

## Example 49

4-(4'-Bromo-biphenyl-4-yl)-2-[2-(4-oxo-4H-benzo[d][1,2,3]triazin-3-yl)-ethyl]-4-oxo-butyric acid

### Intermediate 49A

tert.-Butyl-4-(4'-bromo-biphenyl-4-yl)-4-oxo-2-(tert.-butyloxycarbonyl)-2-[2-(4-oxo-4H-benzo[d][1,2,3]triazin-3-yl)-ethyl]-butanoate

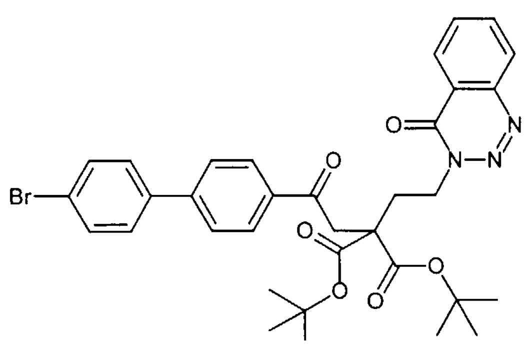

A solution of Di-*tert*.-butyl-2-[2-(4-oxo-4H-benzo[d][1,2,3]triazin-3-yl)-ethyl]-malonate (1.27 g, 3.25 mmol) in 15 mL DMF was added dropwise to a suspension of NaH ( 160 mg, 4.06 mmol) in 5 mL DMF and stirred for 30 min at rt. 2-Bromo-4'-(4'-bromo-phenyl) acetophenone (1.15 g, 3.25 mmol) in 15 ml DMF was added slowly and the resulting mixture was stirred for 2.5 h at rt. The reaction was quenched with saturated $NH_4Cl$ solution, extracted twice with diethyl ether, washed with saturated $NaHCO_3$, water and brine, dried ($Na_2SO_4$) and evaporated. The crude product was purified using flash chromatography (Hexane / Ethyl acetate : 9/1-> 3/1).
Yield: 1.27 g (58 %)
$^1$H-NMR ($D_6$-DMSO): 7.88-8.25 (m, 6H), 7.81 (d, J=8Hz, 2H), 7.7 (s, 4H), 4.39-4.51 (m, 2H), 3,75 (s, 2H), 2.45-2.58 (m, 2H), 1.4 (s, 18H)

**Example 49**

4-(4'-Bromo-biphenyl-4-yl)-2-[2-(4-oxo-4H-benzo[d][1,2,3]triazin-3-yl)-ethyl]-4-oxo-butyric acid

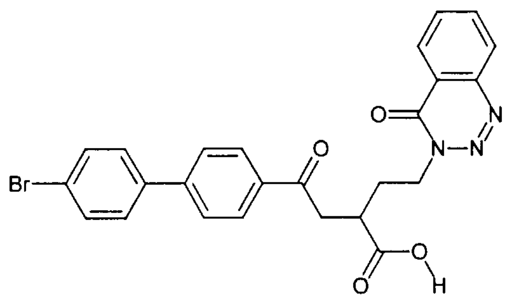

1.24 g (1.87 mmol) of Intermediate 49A was added in one portion to a cooled (0°C) 1:1 mixture of $CH_2Cl_2$ and trifluoroacetic acid (20 mL). The reaction mixture was stirred for 2 h at rt, evaporated and dried under vacuum. The residue was disolved in 20 mL dioxane and heated for 5 h under reflux. The rection mixture was evaporated, the residue triturated with ethylacetate, stirred for 15 min and filtered. The remaining solid was dried under vacuum.
Yield: 0.77 g (78 %)
$^1$H-NMR ($D_6$-DMSO): 12.4 (s, 1H), 7.9-8.3 (m, 6H), 8579 (d, J=8Hz, 2H), 7.72 (s, 4H), 3.28-3.62 (m, 4H), 2.88-3.05 (m,1H), 2.02-2.35 (m, 2H)
700mg of the racemate were separated into pure enantiomers via chiral HPLC.

**Example 50**

First eluting enantiomer A:
Yield: 265mg (38%)
$[\alpha]_D^{20}$(c=0.61g/100ml, THF) = +14.34°

**Example 51**

Slower eluting enantiomer :
Yield: 219mg (31%)
$[\alpha]_D^{20}$(c=0.62 g/100ml, THF) = -14.32°

**Example 52**

4-(4'-methyl[1,1'-biphenyl]-4-yl)-4-oxo-2-{2-[4-oxo-1,2,3-benzotriazin-3(4H)-yl]ethyl}butanoic acid

**Intermediate 52A**

1-(4'-methyl[1,1'-biphenyl]-4-yl)-1-ethanone

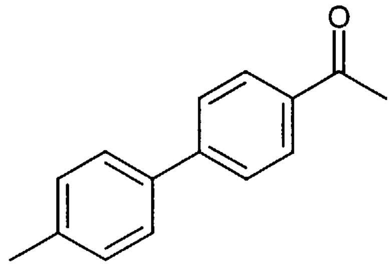

20g (119mmol) 4-methylbiphenyl and 19.81g (149mmol) aluminiumchlorid are dissolved in 120ml dichloromethane. At a temperature below 20°C 9.38g (119mmol) acetylchloride is added dropwise and the reactionmixture is stirred at roomtemperature for 2hrs. The solution is added to a mixture of 200ml ice water and 55ml conc. HCl, the organic phase is separated, dried and concentrated. The residue was purified by chromatography (cyclohexane/ethylacetate=10:1).
Yield: 11.5g (46%).

200 MHz $^1$H-NMR ($CDCl_3$): 2.41, s, 3H; 2.62, s, 3H; 7.29, d, 2H; 7.53, d, 2H; 7.68, d, 2H; 8.02, d, 2H.

**Intermediate 52B**

2-bromo-1-(4'-methyl[1,1'-biphenyl]-4-yl)-1-ethanone

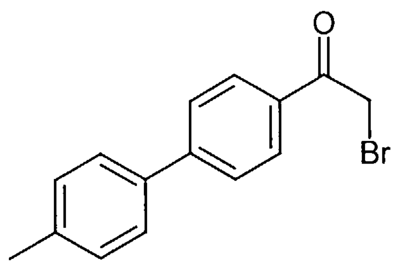

4.76g (22.6mmol) of Intermediate 52A are dissolved in a mixture of 50ml ether and 50ml methanol with gentle heating. The solution is treated with 3.06g (29.4mmol) boronicacidtrimethylester at roomtemperature. 4.16g (26mmol) bromine are added dropwise at 0°C. The reaction mixture is stirred until disappearance of the yellow colour. The solvents are removed in vacuo and the product obtained by triturating with ether.
Yield: 4.69g (72%)

200 MHz $^1$H-NMR ($CDCl_3$): 2.41, s, 3H; 4.47, s, 2H; 7.29, d, 2H; 7.53, d, 2H; 7.70, d, 2H; 8.06, d, 2H.

**Example 52**

4-(4'-methyl[1,1'-biphenyl]-4-yl)-4-oxo-2-{2-[4-oxo-1,2,3-benzotriazin-3(4H)-yl]ethyl}butanoic acid

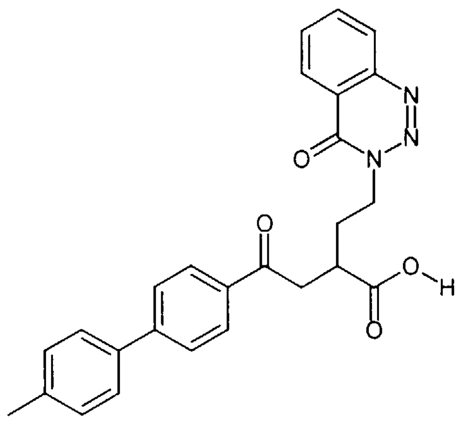

To a suspension of 0.257g (6.42mmol) sodiumhydride (60% in mineral oil) in 20ml DMF under argon is added dropwise a solution of 2.0g (5.14mmol) di(tert-butyl) 2-{2-[4-oxo-1,2,3-benzotriazin-3(4H)-yl]ethyl}malonate in 20ml DMF. After stirring for 30 minutes at room temperature 1.49g (5.14mmol) 2-bromo-1-(4'-methyl[1,1'-biphenyl]-4-yl)-1-ethanone in 20ml DMF are added dropwise. The reaction mixture is stirred at room temperature for 2.5 hrs and poured onto 150ml saturated $NH_4Cl$-solution. The aqueous phase is extracted twice with ethylacetate, the combined organic phases are dried and solvents removed in vacuo. The residue is purified by chromatography (cyclohexan/ethylacetate:10:1), to give 1.87g (61%) di(tert-butyl)2-[2-(4'-methyl[1,1'-biphenyl]-4-yl)-2-oxoethyl]-2-{2-[4-oxo-1,2,3-benzotriazin-3(4H)-yl]ethyl}malonate.

0.46g (0.77mmol) di(tert-butyl)2-[2-(4'-methyl[1,1'-biphenyl]-4-yl)-2-oxoethyl]-2-{2-[4-oxo-1,2,3-benzotriazin-3(4H)-yl]ethyl}malonate is dissolved in a mixture of 4ml dichloromethane and 4ml trifluoroaceticacid and the resulting solution is stirred for 2 hrs at room temperature. 10ml toluene are added and the solvents are removed in vacuo. The residue is taken up in 8ml 1,4-dioxane and the resulting solution refluxed for 6 hrs and stirred at room temperature for 12hrs. The solvent is removed in vacuo and the product obtained by trituration with ethylacetate.
yield: 0.233g (65%)

200 MHz $^1$H-NMR ($CDCl_3$+DMSO): 2.22, m, 1H; 2.40, s, 3H; 2.70-3.02, m, 2H; 3.29, m, 1H; 3.54, m, 1H; 4.63, t, 2H; 7.28, d, 2H; 7.55, d, 2H; 7.65, d, 2H; 7.78-8.04, m, 4H; 8.16, d, 1H; 8.33, d, 1H.

The racemate was separated into the enantiomers via chiral HPLC:

## Example 53

First eluting enantiomer A:
$[\alpha]_D^{20}$(c=0.88 g/100ml, THF) = +15.2°

## Example 54

Second eluting enantiomer B
$[\alpha]_D^{20}$(c=0.93 g/100ml, THF) = +13.2°

## Example 55

### Intermediate 55A

1-(4'-ethyl[1,1'-biphenyl]-4-yl)-1-ethanone

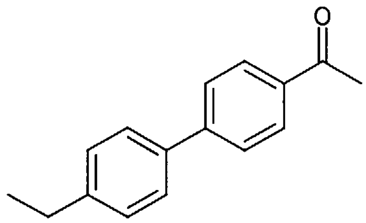

10g (55mmol) 4-ethylbiphenyl and 9.15g (169mmol) aluminiumtrichloride are dissolved in 60ml nitrobenzene. At a temperature below 20°C 4.31g (55mmol) acetylchloride is added dropwise and the reactionmixture is stirred at roomtemperature for 2hrs. The solution is added to a mixture of 80ml ice water and 14ml conc. HCl, the organic phase is separated, dried and concentrated. The product is obtained by trituration with petrolether.
Yield: 8.8g (71%).

200 MHz $^1$H-NMR ($CDCl_3$): 1.29, t, 3H; 2.52, s, 3H; 2.72, quart., 2H; 7.30, d, 2H; 7.58, d, 2H; 7.69, d, 2H; 8.02, d, 2H.

**Intermediate 55B**

2-bromo-1-(4'-ethyl[1,1'-biphenyl]-4-yl)-1-ethanone

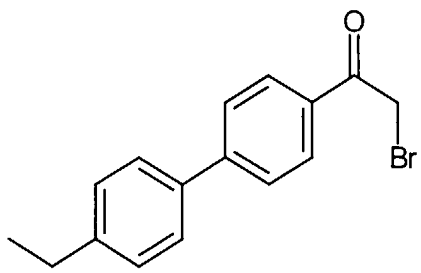

3.45g (15.4mmol) of intermediate 55A are dissolved in a mixture of 70ml methanol and 40ml ethanol with gentle heating. The solution is treated with 2.08g (20mmol) boronicacidtrimethylester at roomtemperature. 2.7g (20mmol) bromine are added dropwise at 0°C. The reaction mixture is stirred until disappearance of the yellow colour. The solvents are removed in vacuo and the product obtained by triturating with ether.
Yield: 3.74g (76%)

200 MHz $^1$H-NMR ($CDCl_3$): 1.29, t, 3H; 2.72, quar., 2H; 4.47, s, 2H; 7.32, d, 2H; 7.57, d, 2H; 7.70, d, 2H; 8.04, d, 2H.

<u>**Example 55**</u>

4-(4'-ethyl[1,1'-biphenyl]-4-yl)-4-oxo-2-{2-[4-oxo-1,2,3-benzotriazin-3(4H)-yl]ethyl}butanoic acid

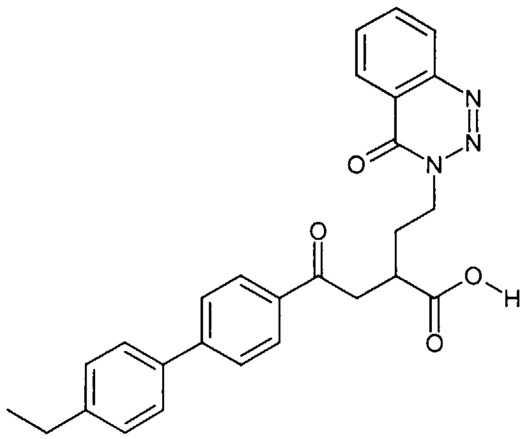

To a suspension of 0.330g (8.25mmol) sodiumhydride (60% in mineral oil) in 20ml DMF under argon is added dropwise a solution of 2.57g (6.6mmol) di(tert-butyl) 2-{2-[4-oxo-1,2,3-benzotriazin-3(4H)-yl]ethyl}malonate in 20ml DMF. After stirring for 30 minutes at room temperature 2.0g (6.60mmol) 2-bromo-1-(4'-ethyl[1,1'-biphenyl]-4-yl)-1-ethanone in 20ml DMF are added dropwise. The reaction mixture is stirred at room temperature for 2.5 hrs and poured onto 150ml saturated $NH_4Cl$-solution. The aqueous phase is extracted twice with ethylacetate, the combined organic phases are dried and solvents removed in vacuo. The residue is purified by chromatography (cyclohexan/ethylacetate: 10:1), to give 3.44g (85%) di(tert-butyl)2-[2-(4'-ethyl[1,1'-biphenyl]-4-yl)-2-oxoethyl]-2-{2-[4-oxo-1,2,3-benzotriazin-3(4H)-yl]ethyl}malonate.
3.44g (5.62mmol) di(tert-butyl)2-[2-(4'-ethyl[1,1'-biphenyl]-4-yl)-2-oxoethyl]-2-{2-[4-oxo-1,2,3-benzotriazin-3(4H)-yl]ethyl}malonate is dissolved in a mixture of 30ml dichloromethane and 30ml trifluoroaceticacid and the resulting solution is stirred for 2 hrs at room temperature. 10ml toluene are added and the solvents are removed in vacuo. The residue is taken up in 60ml 1,4-dioxane and the resulting solution refluxed for 6 hrs and stirred at room temperature for 12hrs. The solvent is removed in vacuo and the product obtained by trituration with ethylacetate.
yield: 1.77g (67%)
200 MHz $^1$H-NMR (DMSO): 1.22, t, 3H; 2.02-2.33, m, 2H; 2.67, quar., 2H; 2.97, m, 1H; 3.26-3.62, m, 2H; 4.53, t, 2H; 7.35, d, 2H; 7.69, d, 2H; 7.82, d, 2H; 7.89-8.13, m, 4H; 8.23, t, 2H; 12.37, s, 1H.

Example 55 was separated into the enantiomers via chiral HPLC:

**Example 56**

First eluting enantiomer A:
$[\alpha]_D^{20}$(c=0.91 g/100ml, THF) = +14.4°

**Example 57**

Second eluting enantiomer B:
$[\alpha]_D^{20}$(c=0.91 g/100ml, THF) = +16.2°

**Example 58**

**Intermediate 58A**

1-(4'-butyl[1,1'-biphenyl]-4-yl)-1-ethanone

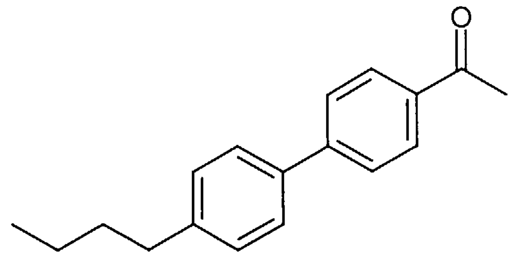

5g (24mmol) 4-butylbiphenyl and 3.96g (30mmol) aluminiumchlorid are dissolved in 30ml nitrobenzene. At a temperature below 20°C 1.87g (30mmol) acetylchloride is added dropwise and the reactionmixture is stirred at roomtemperature for 2hrs. The solution is poured onto icewater, the organic phase is separated, the aqueous phase extracted with ethylacetate, the combined organic phases are washed with water and brine, dried and concentrated. The product is obtained by trituration with petrolether.
Yield: 3.07g (51%).
200 MHz $^1$H-NMR ($CDCl_3$): 0.96, t, 3H; 1.40, m, 2H; 1.65, m, 2H; 2.62, s, 3H; 2.68, t, 2H; 7.30, d, 2H; 7.58, d, 2H; 7.69, d, 2H; 8.02, d, 2H.

**Intermediate 58B**

2-bromo-1-(4'-butyl[1,1'-biphenyl]-4-yl)-1-ethanone

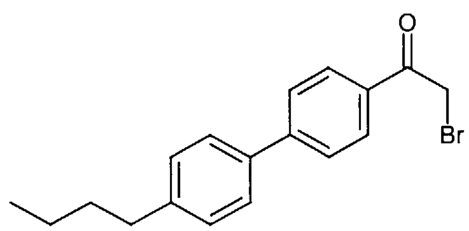

3.07g (12.2mmol) of Intermediate 58A are dissolved in a mixture of 50ml ethanol and 50ml methanol with gentle heating. The solution is treated with 1.64g (15.8mmol) boronicacidtrimethylester at roomtemperature. 2.04g (12.8mmol) bromine are added dropwise at 0°C. The reaction mixture is stirred until disappearance of the yellow colour. The solvents are removed in vacuo and the product obtained by triturating with petrolether.
Yield: 3.02g (75%)
200 MHz $^1$H-NMR ($CDCl_3$): 0.95, t, 3H; 1.30-1.49, m, 2H; 1.57-1.73, m, 2H; 2.68, t, 2H; 4.48, s, 2H; 7.30, d, 2H; 7.55, d, 2H; 7.70, d, 2H; 8.05, d, 2H.

**Example 58**

4-(4'-butyl[1,1'-biphenyl]-4-yl)-4-oxo-2-{2-[4-oxo-1,2,3-benzotriazin-3(4H)-yl]ethyl}butanoic acid

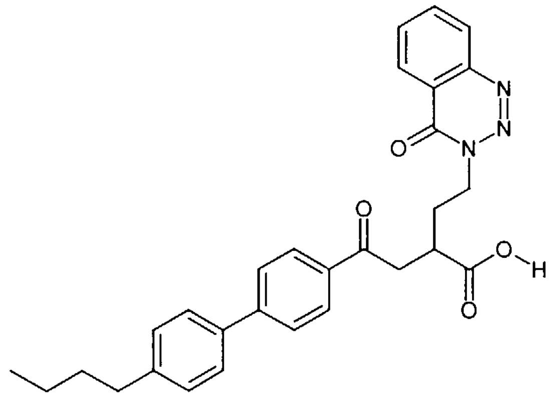

To a suspension of 0.453g (11.3mmol) sodiumhydride (60% in mineral oil) in 20ml DMF under argon is added dropwise a solution of 3.53g (9.06mmol) di(tert-butyl) 2-{2-[4-oxo-1,2,3-benzotriazin-3(4H)-yl]ethyl}malonate in 30ml DMF. After stirring for 30 minutes at room temperature 3.0g (9.06mmol) Intermediate 58B in 30ml DMF are added dropwise. The reaction mixture is stirred at room temperature for 2.5 hrs and poured onto 150ml saturated $NH_4Cl$-solution. The aqueous phase is extracted twice with ethylacetate, the combined organic phases are dried and solvents removed in vacuo. The residue is purified by chromatography (cyclohexan/ethylacetate:10:1). to give 4.46g (77%) of Example 58.

4.46g (77%) di(tert-butyl)2-[2-(4'-butyl[1'1'-biphenyl]-4-yl)-2-oxoethyl]-2-{2-[4-oxo-1,2,3-benzotriazin-3(4H)-yl]ethyl} malonate is dissolved in a mixture of 35ml dichloromethane and 35ml trifluoroaceticacid and the resulting solution is stirred for 2.5 hrs at room temperature. 10ml toluene are added and the solvents are removed in vacuo. The residue is taken up in 70ml 1,4-dioxane and the resulting solution refluxed for 6 hrs and stirred at room temperature for 12hrs. The solvent is removed in vacuo and the product obtained by trituration with ethylacetate.

yield: 1.96g (58%).

200 MHz $^1$H-NMR (DMSO): 0.92, t, 3H; 1.33, m, 2H; 1.60, m, 2H; 2.03-2.30, m, 2H; 2.63, t, 2H; 2.96, m, 1H; 3.23-3.61, m, 2H; 4.54, m, 2H; 7.32, d, 2H; 7.67, d, 2H; 7.80, d, 2H; 7.90-8.13, m, 4H; 8.24, t, 2H; 12.37, s, 1H.

## Claims

**1.** Use of compounds the generalized formula (I):

$$(T)_X A\text{-}B\text{-}D\text{-}E\text{-}CO_2H \qquad \text{(I)}$$

wherein

(a) $(T)_X A$ represents a substituted or unsubstituted aromatic or heteroaromatic moiety selected from the group consisting of:

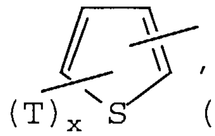 , 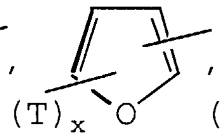 , 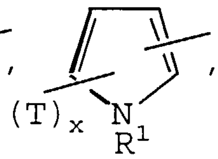 , 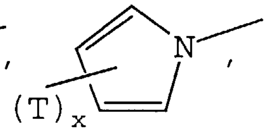 ,

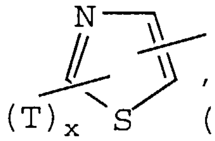 , 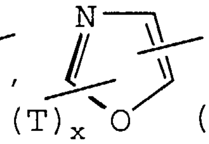 , 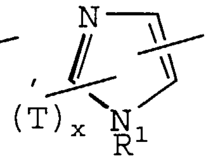 , 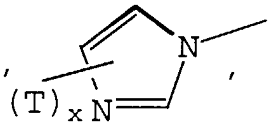 ,

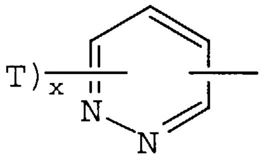 , 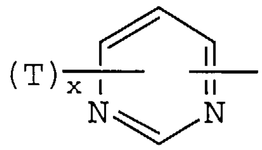 , 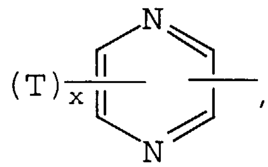 ,

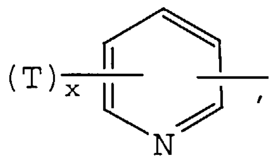 , and 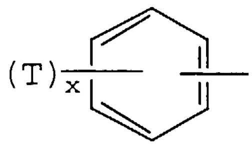

wherein $R^1$ represents H or alkyl of 1 - 3 carbons; and
each T represents a substituent group, independently selected from the group consisting of:

* the halogens -F, -Cl, -Br, and -I;
* alkyl of 1 - 10 carbons;
* haloalkyl of 1 - 10 carbons;
* haloalkoxy of 1 - 10 carbons;
* alkenyl of 2 - 10 carbons;
* alkynyl of 2 - 10 carbons;
* $-(CH_2)_pQ$, wherein
  p is 0 or an integer 1 - 4,
* -alkenyl-Q, wherein
  said alkenyl moiety comprises 2 - 4 carbons, and
* -alkynyl-Q , wherein
  said alkynyl moiety comprises 2 - 7 carbons; and

Q is selected from the group consisting of aryl of 6 - 10 carbons, heteroaryl comprising 4 - 9 carbons and at least one N, O, or S heteroatom, -CN, -CHO, $-NO_2$, $-CO_2R^2$, $-OCOR^2$, $-SOR^3$,$-SO_2R^3$, $-CON(R^4)_2$, $-SO_2N(R^4)_2$, $-C(O)R^2$ , $-N(R^4)_2$, $-N(R^2)COR^2$, $-N(R^2)CO_2R^3$, $-N(R^2)CON(R^4)_2$, $-CHN_4$, $-OR^4$, and $-SR^4$;

wherein

$R^2$ represents H;

alkyl of 1 - 6 carbons;
aryl of 6- 10 carbons;
heteroaryl comprising 4 - 9 carbons and at least one N, O, or S heteroatom; or
arylalkyl in which the aryl portion contains 6 - 10 carbons and the alkyl portion contains 1 - 4 carbons; or
heteroaryl-alkyl in which the heteroaryl portion comprises 4 - 9 carbons and at least one N, O, or S heteroatom and the alkyl portion contains 1 - 4 carbons;

$R^3$ represents alkyl of 1 - 4 carbons;

aryl of 6- 10 carbons;
heteroaryl comprising 4 - 9 carbons and at least one N, O, or S heteroatom; or
arylalkyl in which the aryl portion contains 6 - 10 carbons and the alkyl portion contains 1 - 4 carbons; or
heteroaryl-alkyl in which the heteroaryl portion comprises 4 - 9 carbons and at least one N, O, or S heteroatom and the alkyl portion contains 1 - 4 carbons;

$R^4$ represents H;

alkyl of 1 - 12 carbons;
aryl of 6- 10 carbons;
heteroaryl comprising 4 - 9 carbons and at least one N, O, or S heteroatom;
arylalkyl in which the aryl portion contains 6 - 10 carbons and the alkyl portion contains 1 - 4 carbons;
heteroaryl-alkyl in which the heteroaryl portion comprises 4 - 9 carbons and at least one N, O, or S heteroatom and the alkyl portion contains 1 - 4 carbons;
alkenyl of 2- 12 carbons;
alkynyl of 2- 12 carbons;
$-(C_qH_{2q}O)_rR^5$ wherein q is 1-3; r is 1 - 3; and $R^5$ is H provided q is greater than 1, or alkyl of 1 - 4 carbons, or phenyl;
alkylenethio terminated with H, alkyl of 1-4 Carbons, or phenyl;
alkyleneamino terminated with H, alkyl of 1-4 carbons, or phenyl;
$-(CH_2)_sX$ wherein s is 1 - 3 and X is halogen;
$-C(O)OR^2$; or
$-C(O)R^2$;

and with the provisos that a) when two $R^4$ groups are situated on a nitrogen, they may be joined by a bond to form a heterocycle, and

b) unsaturation in a moiety which is attached to Q or which is part of Q is separated from any N, O, or S of Q by at least one carbon atom, and

x is 0, 1, or 2;

(b) B represents a bond or an optionally substituted aromatic or heteroaromatic ring containing 0-2 substituents T, which substituents T may independently have the meaning specified under (a), the B rings being selected from the group consisting of:

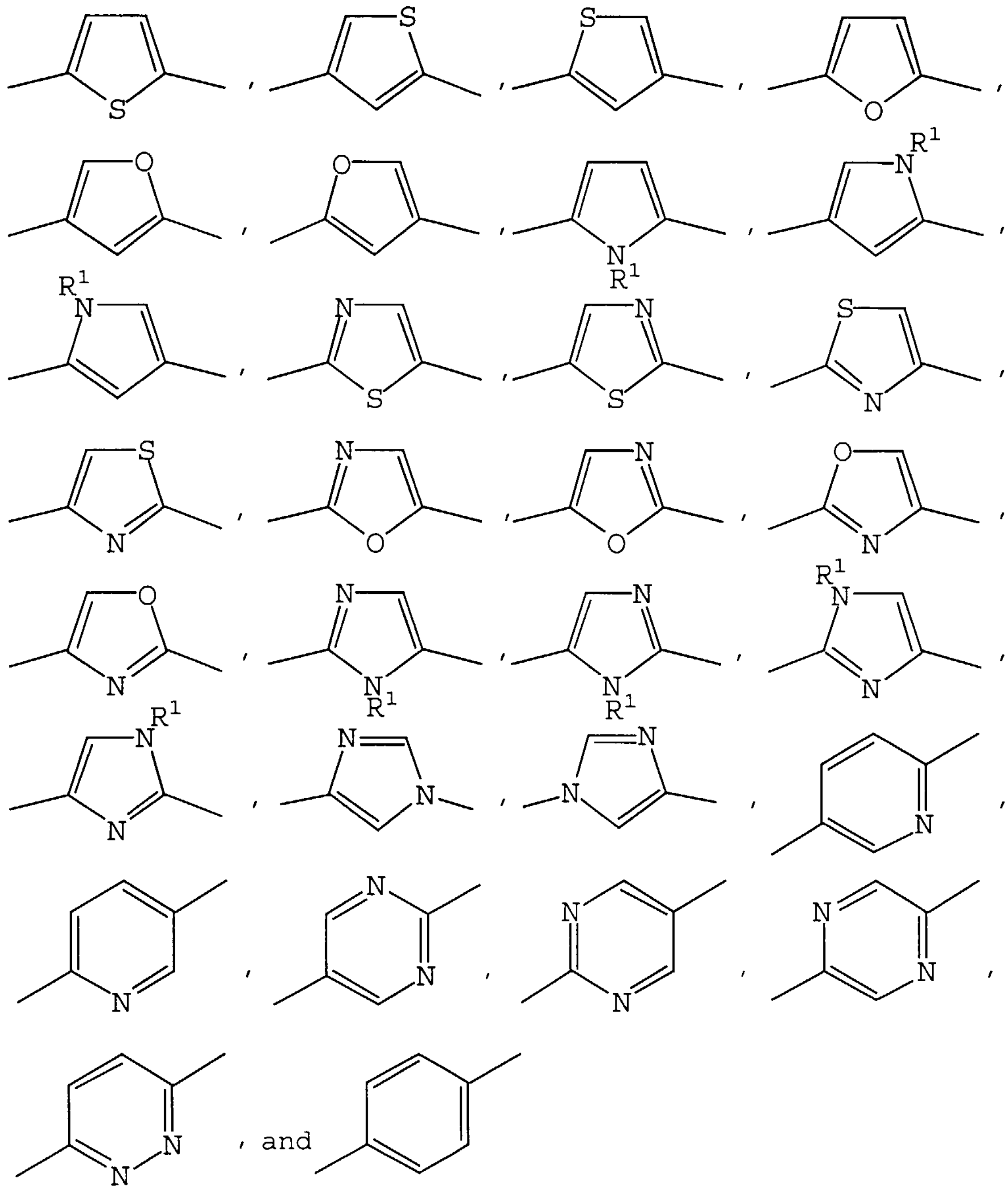

, and

wherein $R^1$ is as defined above and each $R^1$ may be the same or different:

(c) D represents >C=O , or

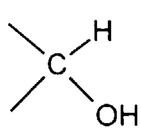

,

(d) E represents a chain of n carbon atoms bearing m substituents $R^6$, wherein said $R^6$ groups are independent substituents, or constitute spiro or nonspiro rings in which a) two groups $R^6$ are joined, and taken together with the chain atom(s) to which said two $R^6$ group(s) are attached, and any intervening chain atoms, constitute

a 3 - 7 membered ring, or b) one group $R^6$ is joined to the chain on which said one group $R^6$ resides, and taken together with the chain atom(s) to which said $R^6$ group is attached, and any intervening chain atoms, constitutes a 3 - 7 membered ring; and wherein

n is 2 or 3;

m is an integer of 1 - 3;

each group $R^6$ is independently selected from the group consisting of:

* fluorine;
* hydroxyl, with the proviso that a single carbon may bear no more than one hydroxyl substituent
* alkyl of 1 - 10 carbons;
* aryl of 6- 10 carbons;
* heteroaryl comprising 4 - 9 carbons and at least one N, O, or S heteroatom;
* arylalkyl wherein the aryl portion contains 6 - 10 carbons and the alkyl portion contains 1 - 8 carbons;
* heteroaryl-alkyl wherein the heteroaryl portion comprises 4 - 9 carbons and at least one N, O, or S heteroatom, and the alkyl portion contains 1 - 8 carbons;
* alkenyl of 2- 10 carbons;
* aryl-alkenyl wherein the aryl portion contains 6 - 10 carbons and the alkenyl portion contains 2 - 5 carbons;
* heteroaryl-alkenyl wherein the heteroaryl portion comprises 4 - 9 carbons and at least one N, O, or S heteroatom and the alkenyl portion contains 2 -5 carbons;
* alkynyl of 2- 10 carbons;
* aryl-alkynyl wherein the aryl portion contains 6 - 10 carbons and the alkynyl portion contains 2 - 5 carbons;
* heteroaryl-alkynyl wherein the heteroaryl portion comprises 4 - 9 carbons and at least one N, O, or S heteroatom and the alkynyl portion contains 2 - 5 carbons;
* $-(CH_2)_tR^7$ wherein

  t is 0 or an integer of 1 - 5; and
  $R^7$ is selected from the group consisting of

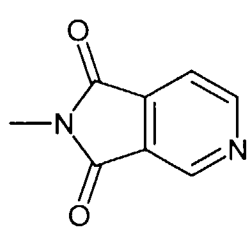

,

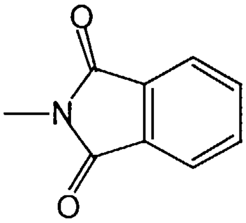 , 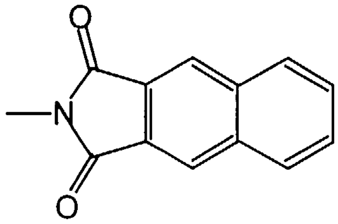 , 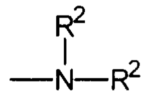 , 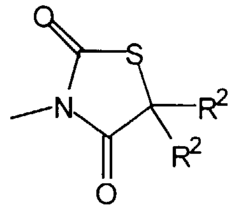 ,

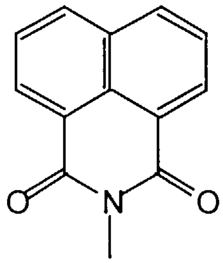 , 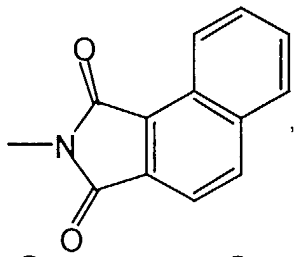 , 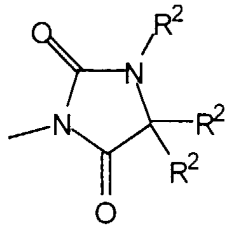 , 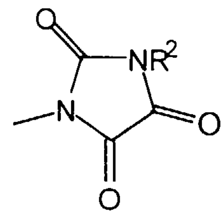 ,

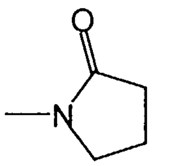 , 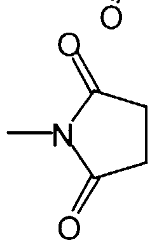 , 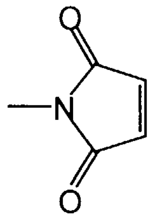 , 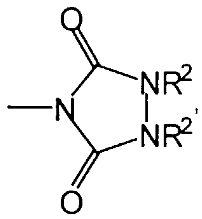 , 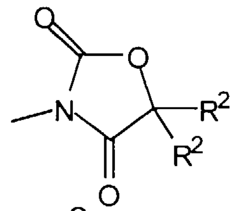 ,

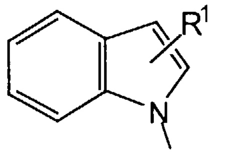 , 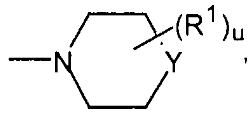 , 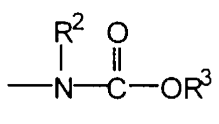 , 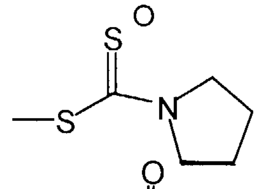 ,

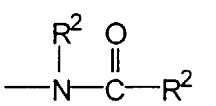 , 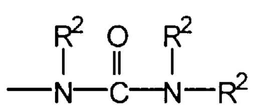 , 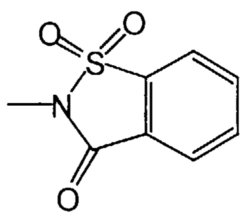 , 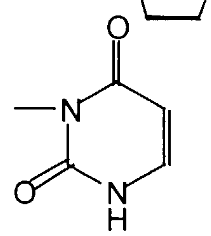 ,

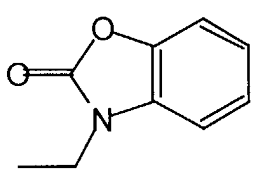 , 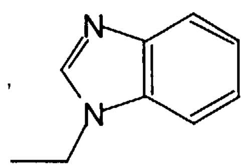 , 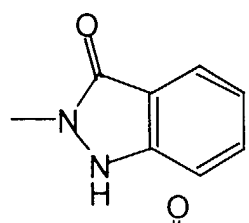 , 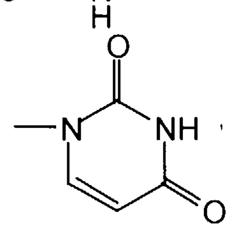 ,

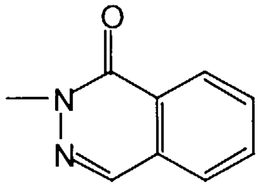 , 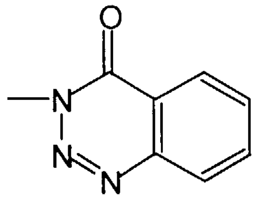 , 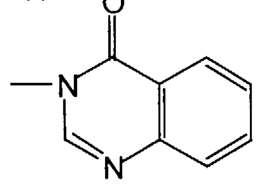 ,

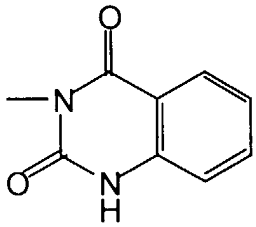 , 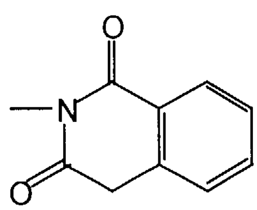 , 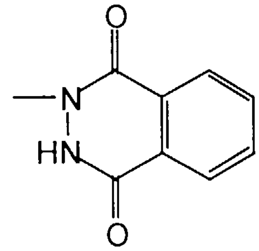 ,

and corresponding heteroaryl moieties in which the aryl portion of an aryl-containing $R^7$ group comprises 4 - 9 carbons and at least one N, O, or S heteroatom;

wherein

Y represents O or S;
$R^1$, $R^2$, and $R^3$ are as defined above and each $R^1$, $R^2$ or $R^3$ may be the same or different; and
u is 0, 1, or 2; and

* $-(CH_2)_vZR^8$ wherein

v is 0 or an integer of 1 to 4; and
Z represents

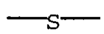 , 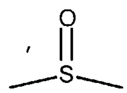 , 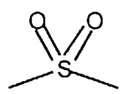 , 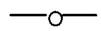 , 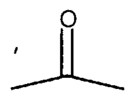 , or 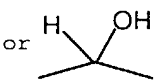 ;

$R^8$ is selected from the group consisting of:

alkyl of 1 to 12 carbons;
aryl of 6 to 10 carbons;
heteroaryl comprising 4 - 9 carbons and at least one N, O, or S heteroatom;
arylalkyl wherein the aryl portion contains 6 to 10 carbons and the alkyl portion contains 1 to 4 carbons;
heteroaryl-alkyl wherein the aryl portion comprises 4 - 9 carbons and at least one N, O, or S heteroatom and the alkyl portion contains 1 - 4 carbons;
$-C(O)R^9$ wherein $R^9$ represents alkyl of 2 - 6 carbons, aryl of 6 - 10 carbons, heteroaryl comprising 4 - 9 carbons and at least one N, O, or S heteroatom, or arylalkyl in which the aryl portion contains 6 - 10 carbons or is heteroaryl comprising 4 - 9 carbons and at least one N, O, or S heteroatom, and the alkyl portion contains 1 - 4 carbons;
and with the provisos that

- when $R^8$ is $-C(O)R^9$ , Z is S or O;
- when Z is O, $R^8$ may also be $-(C_qH_{2q}O)_rR^5$ wherein q, r, and

$R^5$ are as defined above; and

* $-(CH_2)_wSiR^{10}_3$ wherein

w is an integer of 1 to 3; and
$R^{10}$ represents alkyl of 1 to 2 carbons;

and with the proviso that

- aryl or heteroaryl portions of any of said T or $R^6$ groups optionally may bear up to two substituents selected from the group consisting of $-(CH_2)_yC(R^4)(R^3)OH$, $-(CH_2)_yOR^4$, $-(CH_2)_ySR^4$, $-(CH_2)_yS(O)R^4$, $-(CH_2)_yS(O)_2R^4$, $-(CH_2)_ySO_2N(R^4)_2$, $-(CH_2)_yN(R^4)2$, $-(CH_2)_yN(R^4)COR^3$, $-OC(R^4)_2O-$ in which both oxygen atoms are connected to the aryl ring, $-(CH_2)_yCOR^4$, $-(CH_2)_yCON(R^4)_2$,$-(CH_2)_yCO_2R^4$, $-(CH_2)_yOCOR^4$, -halogen, -CHO, $-CF_3$, $-NO_2$, -CN, and-$R^3$, wherein

y is 0 - 4; and
$R^3$ and $R^4$ are defined as above, and each $R^3$ or $R^4$ may be the same or different; and any two $R^4$ which are attached to one nitrogen may be joined to form a heterocycle;

and pharmaceutically acceptable salts and prodrugs thereof for the manufacturing of drugs for the treatment and prevention of cerebral diseases.

European Patent Office

# PARTIAL EUROPEAN SEARCH REPORT

which under Rule 45 of the European Patent Convention shall be considered, for the purposes of subsequent proceedings, as the European search report

Application Number
EP 99 10 3723

## DOCUMENTS CONSIDERED TO BE RELEVANT

| Category | Citation of document with indication, where appropriate, of relevant passages | Relevant to claim | CLASSIFICATION OF THE APPLICATION |
|---|---|---|---|
| X | WO 98 26773 A (BOCAN THOMAS MICHAEL ANDREW ;PETERSON JOSEPH THOMAS JR (US); WARNE) 25 June 1998 (1998-06-25)<br>* abstract *<br>* page 4, line 14 - line 27 *<br>* page 15 *<br>* page 16, line 1 - page 17, line 16 *<br>* page 21, line 1 - page 31, line 15; claims 1-5 *<br>--- | 1 | A61K31/19<br>A61K31/40<br>A61K31/53<br>A61K31/435<br>A61K31/24<br>A61K31/425<br>A61K31/42<br>A61K31/50<br>A61K31/195<br>A61K31/44 |
| X | WO 98 09940 A (PURCHASE CLAUDE FORSEY JR ;WARNER LAMBERT CO (US); ROTH BRUCE DAVI) 12 March 1998 (1998-03-12)<br>* abstract *<br>* page 1, line 1 - line 20 *<br>* page 5, line 27 - page 9, line 16 *<br>* page 13, line 14 - page 17, line 21 *<br>* page 24, line 14 - page 26, line 7 *<br>* page 71, line 5 - line 24; claims 8-17,38-40,53-55; example 7 *<br>---<br>-/-- | 1 | |
| | | | TECHNICAL FIELDS SEARCHED |
| | | | A61K |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 11 August 1999 | Hoff, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 99 10 3723

Claim(s) searched completely:
-

Claim(s) searched incompletely:
1

Reason for the limitation of the search:

Present claim 1 relates to an extremely large number of possible compounds. Support within the meaning of Article 84 EPC and/or disclosure within the meaning of Article 83 EPC is to be found, however, for only a very small proportion of the compounds claimed. In the present case, the claims so lack support, and the application so lacks disclosure, that a meaningful search over the whole of the claimed scope is impossible. Consequently, the search has been carried out for those parts of the claims which appear to be supported and disclosed, namely those parts relating to the compounds mentioned in table 1, in the examples 44 to 58, and closely related homologous compounds such as mentioned in the description at page 22.

European Patent Office

# PARTIAL EUROPEAN SEARCH REPORT

Application Number
EP 99 10 3723

| Category | DOCUMENTS CONSIDERED TO BE RELEVANT<br>Citation of document with indication, where appropriate, of relevant passages | Relevant to claim | CLASSIFICATION OF THE APPLICATION |
|---|---|---|---|
| D,X | WO 96 15096 A (SCHNEIDER STEPHAN ;BENZ GUENTER H H H (DE); DIXON BRIAN RICHARD (U) 23 May 1996 (1996-05-23)<br>* abstract *<br>* page 10, line 29 - page 11, line 6; claims 1-23; examples; table XXVIII *<br>--- | 1 | |
| D,X | WO 98 22436 A (BAYER AG)<br>28 May 1998 (1998-05-28)<br>* abstract *<br>* page 16, line 20 - line 34; claims; examples *<br>--- | 1 | |
| D,X | WO 97 43239 A (BRITTELLI DAVID R ;ZANDT MICHAEL C VAN (US); DIXON BRIAN R (US); B)<br>20 November 1997 (1997-11-20)<br>* abstract *<br>* page 10, line 19 - page 11, line 11; claims; examples *<br>--- | 1 | TECHNICAL FIELDS SEARCHED |
| D,X | WO 97 43245 A (DIXON BRIAN R ;BAYER AG (US); CHEN JINSHAN (US))<br>20 November 1997 (1997-11-20)<br>* abstract *<br>* page 9, line 20 - page 10, line 10; claims; examples *<br>--- | 1 | |
| D,X | WO 97 43240 A (HARTSOUGH DAVID S ;BAYER AG (US); POPP MARGARET A (US); SCOTT WILL)<br>20 November 1997 (1997-11-20)<br>* abstract *<br>* page 9, line 5 - line 20; claims; examples *<br>---<br>-/-- | 1 | |

EPO FORM 1503 03.82 (P04C10)

European Patent Office

# PARTIAL EUROPEAN SEARCH REPORT

Application Number
EP 99 10 3723

## DOCUMENTS CONSIDERED TO BE RELEVANT

| Category | Citation of document with indication, where appropriate, of relevant passages | Relevant to claim | CLASSIFICATION OF THE APPLICATION |
|---|---|---|---|
| D,X | WO 97 43247 A (BAYER AG ;WOLANIN DONALD J (US)) 20 November 1997 (1997-11-20)<br>* abstract *<br>* page 13, line 15 - page 14, line 3; claims; examples *<br>--- | 1 | |
| X | EP 0 465 879 A (VYZK USTAV FARM BIOCHEM SP) 15 January 1992 (1992-01-15)<br>* examples; tables I,II *<br>--- | 1 | |
| X | CHILD R G ET AL: "A NEW NON-STEROIDAL ANTI-INFLAMMATORY ANALGESIC: GAMMA-OXO (1,1' -BIPHENYL)-4-BUTANOIC ACID (FENBUFEN). CHEMISTRY AND ACTIVITY OF ANALOGS"<br>ARZNEIMITTEL FORSCHUNG. DRUG RESEARCH, vol. 30, no. 4A,<br>1 January 1980 (1980-01-01), pages 695-702, XP002043280<br>ISSN: 0004-4172<br>* the whole document *<br>----- | 1 | TECHNICAL FIELDS SEARCHED |

EPO FORM 1503 03.82 (P04C10)